# EUROPÄISCHE PATENTSCHRIFT

(11) **EP 3 050 585 B1**
(45) Veröffentlichungstag und Bekanntmachung des Hinweises auf die Patenterteilung: **10.04.2019**
(21) Anmeldenummer: 15152703.3
(22) Anmeldetag: 27.01.2015
(51) Int. Cl.: A61M 5/142, F04B 7/04, A61M 5/14

(54) **Dosiergerät zur Abgabe eines Fluids unter aseptischen Bedingungen**
Dosing device for dispensing a fluid under aseptic conditions
Appareil de dosage destiné à la distribution d'un fluide dans des conditions aseptiques

(43) Veröffentlichungstag der Anmeldung: 03.08.2016
(73) Patentinhaber: Idorsia Pharmaceuticals Ltd, 4123 Allschwil (CH)
(72) Erfinder: Weibel, Ludwig Daniel, 9104 Waldstatt (CH); Wyler, Samuel, 9030 Abtwil (CH)
(74) Vertreter: Hepp Wenger Ryffel AG

(56) Entgegenhaltungen:
- EP-A1- 0 701 061
- EP-A1- 2 628 494
- WO-A1-2014/090745
- WO-A1-2014/207532
- GB-A- 1 508 665
- US-A- 1 516 032
- US-A- 5 513 779
- US-A1- 2011 073 620

## Beschreibung

Die Erfindung betrifft ein Dosiergerät zur Abgabe eines Fluids unter aseptischen Bedingungen gemäss dem Oberbegriff des Anspruchs 1.

Bei der Verabreichung flüssiger Formulierungen pharmazeutischer Wirkstoffe an einen Patienten ist es in den meisten Fällen erforderlich, wohl definierte Volumina abzugeben. Häufig müssen die Arzneimittel dabei in den Körper des Patienten injiziert werden. Zur parenteralen Injektion kommen hierzu Injektionsspritzen, Arzneimittelstifte (sogenannte Pens) oder Arzneimittelpumpen zum Einsatz.

In diesem Zusammenhang finden zur einmaligen Verwendung vorgesehene Injektionsspritzen aufgrund ihres geringen Preises und der grossen Verfügbarkeit breite Anwendung. Die Verwendung solcher Spritzen erfordert jedoch geschultes Personal für eine fehlerfreie Applikation. So muss die flüssige Formulierung gegebenenfalls aus einer entsprechenden Ampulle aufgezogen werden, wobei unter anderem auf eine ausreichende Blasenfreiheit und sterile Bedingungen zu achten ist. Dies ist insbesondere bei Präparaten problematisch, die über eine längere Zeitdauer verabreicht werden müssen. So müssen beispielsweise Patienten, die unter Diabetes leiden, sich oft mehrmals täglich unter stark wechselnden Bedingungen selbst eine Insulindosis verabreichen.

Injektionsspritzen werden daher in solchen Fällen oft durch so genannte Arzneimittel-Pens ersetzt. Diese haben den Vorteil, dass sie mehrere Dosen eines Präparates nacheinander abgeben können. Hierzu verfügen sie über einen auswechselbaren Vorratsbehälter (z.B. eine Ampulle), welcher vorgefüllt und steril ausgeliefert wird und in den Arzneimittel-Pen einsetzbar ist. Jedoch muss bei einem Arzneimittel-Pen die Applikation auch durch den Patienten selbst erfolgen. Dies erfordert eine Reihe von Fähigkeiten bezüglich der richtigen Anwendung und Dosierung. Ferner ist stets auf Sauberkeit und Hygiene zu achten.

Arzneimittelpumpen sind hingegen dazu geeignet, über einen längeren Zeitraum am Körper eines Patienten angebracht zu werden und ein Präparat kontinuierlich bzw. nach einem individuell vorgegebenen Schema zu verabreichen. Derartige Arzneimittelpumpen umfassen typischerweise einen Behälter für die flüssige Formulierung sowie eine Fördervorrichtung (beispielsweise eine Pumpe), welche das Arzneimittel zu einem Anschluss der Vorrichtung oder zu einem Injektionssystem fördert. Das Injektionssystem kann in gewissen Fällen eine Verweilkanüle umfassen, die während des gesamten Verabreichungszeitraumes im Körper des Patienten verbleibt.

So zeigt die US 2005/0177111 A1 beispielsweise eine Mikroinfusionspumpe zur kontrollierten Abgabe einer Medikation an einen Patienten. Die besagte Pumpe ist im Wesentlichen als Spritzenpumpe ausgestaltet und verfügt über einen integrierten Antriebsmechanismus. Die Verabreichung der flüssigen Formulierung von der Pumpe an den Patienten erfolgt über einen flexiblen Infusionsschlauch. Die Pumpe, welche beispielsweise zur Verabreichung von Insulin an einen Diabetiker eingesetzt werden kann, ist kompakt genug, um am Körper des Patienten angebracht zu werden. Zudem verfügt die Vorrichtung über einen Schaltkreis zur Steuerung des Pumpenantriebs sowie über einen Drucksensor zur Messung des auf den Spritzenkolben ausgeübten Druckes und einen weiteren Sensor zur Bestimmung der Position des Spritzenkolbens.

Die WO 2014/090745 A1 offenbart eine medizinische Pumpe zur Abgabe einer Flüssigkeit, beispielsweise einer Insulinlösung, an einen Patienten. Die Pumpe arbeitet nach dem Doppelkolbenprinzip und ermöglicht eine Reihe von Anwendungen, wie das Mischen zweier Flüssigkeiten oder die Analyse einer vom Patienten entnommenen Probe.

Die WO 2014/207532 A1 befasst sich mit einem volumetrischen Dosiergerät in kompakter Bauweise, welches unter anderem zur Abgabe von Medikamentenlösungen an Patienten unter aseptischen Bedingungen geeignet ist. Das Gerät basiert auf einer Fördervorrichtung, die als Doppelkolbenpumpe ausgeführt ist. Eine hauptsächliche Anwendung liegt in der Dosierung kleiner Flüssigkeitsvolumina, beispielsweise durch ein Implantat oder durch einen auf der Körperoberfläche getragenen Patch.

Derartige Arzneimittelpumpen haben den Vorteil, dass durch sie ohne aktive Beteiligung des Patienten ein Präparat kontinuierlich an ihn abgegeben werden kann. Sie unterscheiden sich damit grundsätzlich von Injektionsspritzen und Arzneimittel-Pens mit manuell durchgeführten Einzelabgaben. Allerdings ist oft die gewünschte Zuverlässigkeit der Pumpe nicht mit ausreichender Sicherheit gewährleistet, oder die Genauigkeit der abgegebenen Arzneimittelmenge entspricht nicht den regulatorischen Vorgaben. Zudem kann durch Arzneimittelpumpen in der Regel nur eine einzige flüssige Formulierung in mehreren Dosen abgegeben werden. Ferner erfolgt die Abgabe eines Präparates durch eine Arzneimittelpumpe in der Regel nach einem festgelegten Schema ohne den aktuellen Zustand des Patienten zu berücksichtigen. So wäre es beispielsweise bei Patienten mit Diabetes wünschenswert, die Insulinabgabe unter einer konstanten Überwachung des Blutzuckerspiegels durchzuführen und diese laufend daran anzupassen.

Aufgrund der lebenserhaltenden Funktion der Abgabe gewisser Arzneimittel bestehen zudem erhöhte Anforderungen an die Zuverlässigkeit einer Arzneimittelpumpe im Betrieb. Da derartige Vorrichtungen über längere Zeit am Körper eines Patienten getragen werden, besteht ferner das Bedürfnis, eine Arzneimittelpumpe möglichst robust und kompakt zu gestalten. Aufgrund der erforderlichen Sterilität ist darüber hinaus zumindest eine teilweise Ausführung als Wegwerfeinheit wünschenswert. Entsprechend besteht das Bedürfnis, eine möglichst einfache und preisgünstige Konstruktion bereit zu stellen.

Allerdings stellt sich die Problematik des exakten Dosierens nicht nur bei der aseptischen Verabreichung flüssiger Pharmazeutika an Patienten, sondern auch beim Befüllen von Ampullen, Beuteln oder Spritzen in der pharmazeutischen Produktion. In den dort eingesetzten Abfüllanlagen kommen hierzu beispielsweise Peristaltikpumpen zum Einsatz. Obwohl derartige Pumpen bedingt durch ihr Wirkprinzip einen hohen Grad an Sterilität gewährleisten, bringt deren Verwendung doch eine Reihe von Nachteilen mit sich. So ist die Lebensdauer des Schlauches bei einer Peristali tikpumpe konstruktionsbedingt beschränkt. Zudem kann ein Abrieb des Schlauches das Fördergut verunreinigen, was eine potentielle Gefährdung für den Patienten darstellt. Nicht zuletzt unterliegt auch die erreichbare Dosiergenauigkeit bei derartigen Pumpen gewissen Einschränkungen.

Es ist daher eine Aufgabe der Erfindung, die Nachteile im Stand der Technik zu überwinden. Insbesondere ist es eine Aufgabe, ein vielseitig einsetzbares Dosiergerät zur Abgabe eines Fluids unter aseptischen Bedingungen bereitzustellen, welches sowohl zuverlässig als auch robust, und komfortabel in der Handhabung, ist. Das Gerät soll sowohl mobil als auch stationär einsetzbar sein. Zudem soll das Dosiergerät eine möglichst hohe Patientensicherheit gewährleisten, eine einfache, kompakte Konstruktion aufweisen und kostengünstig herstellbar sein.

Diese Aufgaben werden durch Dosiergeräte gelöst, welche die Merkmale in den Ansprüchen 1 und 21 aufweisen.

Das besagte Dosiergerät umfasst eine von zumindest einem Förderantrieb angetriebene Fördervorrichtung zur Förderung eines Fluids aus dem Innenraum eines Behälters. Das Fluid ist dabei mittels der Fördervorrichtung vom Behälter zu einer Abgabeöffnung förderbar. Die Fördervorrichtung umfasst einen Zylinder mit wenigstens einer Ansaugöffnung und wenigstens einer Auslassöffnung an einer Zylinderinnenwand sowie einen ersten Kolben und einen zweiten Kolben. Der erste Kolben und der zweite Kolben sind dabei innerhalb des Zylinders in Längsrichtung verschiebbar gelagert. Ferner begrenzen der erste Kolben und der zweite Kolben zwischen ihren Stirnseiten gemeinsam mit einem Abschnitt der Zylinderinnenwand ein variables Fluidvolumen.

Ein derartiges Dosiergerät mit zwei Kolben in einem Zylinder erlaubt vielfältige Möglichkeiten. Darüber hinaus kann das Dosiergerät ventillos ausgeführt sein, wodurch sich eine besonders einfache Bauweise ergibt. Dies erlaubt nicht nur ein erfindungsgemässes Dosiergerät besonders kostengünstig herzustellen, sondern erhöht auch dessen Zuverlässigkeit bei längerem Betrieb. Zudem ermöglicht eine Konstruktion ohne Ventile einen sehr hohen Miniaturisierungsgrad, wie er beispielsweise bei tragbaren Insulinabgabesystemen erforderlich ist.

Die Ansaugöffnung und die Auslassöffnung können in Längsrichtung versetzt am Zylinder angeordnet sein. Dies ermöglicht den Einsatz von Kolben, bei welchen die Zylinderlängsachse senkrecht zur Stirnseite verläuft. Dadurch wird der Aufbau der Fördervorrichtung weiter vereinfacht.

Die Ansaugöffnung kann mit dem Innenraum eines Behälters und die Auslassöffnung mit der Abgabeöffnung in Fluidverbindung bringbar sein. Dadurch lässt sich durch die Fördervorrichtung das Fluid vom Innenraum eines Behälters direkt zur Abgabeöffnung transportieren.

Das Dosiergerät kann einen Drucksensor aufweisen, welcher vorzugsweise am Zylinder, insbesondere in Längsrichtung auf Höhe der Auslassöffnung, angeordnet ist und direkt den Fluiddruck misst. Durch das Vorhandensein eines derartigen Drucksensors kann der Abgabeprozess des Fluids in Echtzeit überwacht und damit die tatsächliche Verabreichung eines Medikamentes an einen Patienten sichergestellt werden. Ferner kann damit nicht nur ein Überdruck in den Fluidführenden Systemen vermieden werden, sondern auch ein unbeabsichtigtes Austreten von Fluiden, sowie mögliche durch Überdruck herbeigeführte Verletzungen eines Patienten.

Das Dosiergerät kann zusätzlich zumindest einen, insbesondere teilweise kollabierbaren, Behälter mit einem Innenraum umfassen. In dieser Ausführung bildet das Dosiergerät eine kompakte Abgabeeinheit, mit welcher beispielsweise ein Fluid über eine längere Zeitdauer an einen Patienten abgegeben werden kann.

Eine, zumindest teilweise, kollabierbare Ausgestaltung des Behälters hat den Vorteil, dass keine Belüftungsöffnungen vorhanden sein müssen, um zum Ausgleich eines aufgrund der Entnahme des Fluids entstehenden Unterdrucks durch nachströmende Luft in den Behälter auszugleichen. Zudem lassen sich kollabierbare Behälter, wie zum Beispiel Beutel, flach ausbilden, was insbesondere bei tragbaren Dosiergeräten eine platzsparende Anordnung ergibt. Allerdings kann der Behälter auch starr mit einem Schleppkolben, welcher wahlweise mit einer Druckfeder vorgespannt sein kann, ausgeführt sein.

Ein derartiges Dosiergerät kann zusätzlich eine Füllöffnung umfassen, welche durch Verschieben des ersten Kolbens und des zweiten Kolbens innerhalb des Zylinders mit der Ansaugöffnung in Fluidkommunikation bringbar ist. Dies ermöglicht das Befüllen des Behälters durch die Fördervorrichtung hindurch, womit weitere Füllzuleitungen überflüssig sind. Auf diese Weise kann der leere Behälter beispielsweise in das Dosiergerät eingesetzt werden, und erst vor Anwendung des Dosiergerätes durch einen Benutzer befüllt werden.

Dabei können die Füllöffnung und die Ansaugöffnung in Längsrichtung versetzt am Zylinder angeordnet sein. Bei dieser Konfiguration kann die Fördervorrichtung in Bezug auf den für die Befüllung erforderlichen Fluidweg auch die Funktion eines Ventiles erfüllen.

Für einen optimalen Füllprozess kann die Füllöffnung mit einem Kopplungsmittel zum Ankoppeln einer Fluidquelle, insbesondere einer Luer-Kupplung, in Fluidkommunikation bringbar sein. Mit Vorteil ist dabei eine Ventilvorrichtung fluidmässig zwischen der Füllöffnung und dem Kopplungsmittel angeordnet, wobei es sich dabei bevorzugt um ein Schnabelventil handelt, welches die Füllöffnung gegen einen Fluidstrom von der Fördervorrichtung nach aussen sperrt. Ein derart ausgeführtes Dosiergerät lässt sich an gängige Behältnisse mit flüssigen pharmazeutischen Formulierungen koppeln. Allerdings kann die Füllöffnung auch ein Septum aufweisen, welches mit einer Füllkanüle penetrierbar ist und sich nach dem Entfernen der Kanüle wieder fluiddicht verschliesst.

Es versteht sich, dass auch vorgefüllte Behälter zum Einsatz kommen können, welche im Lieferzustand bereits mit dem Fluid gefüllt und an das Dosiergerät gekoppelt bzw. koppelbar sind.

Die Fördervorrichtung kann zumindest zwei Ansaugöffnungen umfassen, welche in Längsrichtung versetzt am Zylinder angeordnet sind. Durch das Vorhandensein mehrerer Ansaugöffnungen lassen sich wahlweise verschiedene Fluide durch ein und dasselbe Dosiergerät abgeben. Dies kann entweder wahlweise separat oder durch Mischen der beiden Fluide durch die Fluidfördervorrichtung erfolgen.

So kann beispielsweise jede Ansaugöffnung mit dem Innenraum eines separaten, ihm zugeordneten, insbesondere teilweise kollabierbaren, Behälters in Fluidverbindung bringbar sein. Denkbar ist beispielsweise, dass sich in den beiden Behältern flüssige Formulierungen desselben Wirkstoffs in unterschiedlichen Konzentrationen befinden. Dadurch ist es möglich, einem Patienten ein Medikament in einem sehr breiten Dosierungsbereich zuzuführen. Allerdings ist es auch vorstellbar, dass in den beiden Behältern zwei unterschiedliche flüssige Formulierungen mit unterschiedlichen Wirkstoffen vorhanden sind. Dies erlaubt es, einem Patienten situativ entweder den einen oder den anderen Wirkstoff zu verabreichen. Ein weiteres Anwendungsbeispiel eines derartigen Dosiergerätes ist die kontinuierliche Abgabe einer Kochsalzlösung aus einem ersten Behälter an einen Patienten, um einen zuvor gelegten Katheter-Zugang offen zu halten. Aus einem zweiten Behälter kann bei einer solchen Anwendung eine Wirkstofflösung bedarfsweise über den Katheter an den Patienten abgegeben werden.

Allerdings können auch zumindest zwei Ansaugöffnungen jeweils mit einem separat abgetrennten Innenraum eines Behälters in Fluidverbindung bringbar sein, wobei die separat abgetrennten Innenräume derart ausgestaltet, sind, dass wenigstens zwei Fluide voneinander getrennt im Behälter aufnehmbar sind. Ein solcher Mehrkammerbehälter ermöglicht das Bereitstellen eines erfindungsgemässen Dosiergerätes, welches die zuvor beschriebenen Anwendungsmöglichkeiten bietet, jedoch eine kompaktere und günstigere Bauweise aufweist.

Sowohl ein Mehrkammerbehälter als auch mehrere Einkammerbehälter können bei pharmazeutischen Wirkstoffen vorteilhaft oder fallweise sogar erforderlich sein, welche sich in Lösung nicht über einen längeren Zeitraum lagern lassen. Derartige Wirkstoffe werden oft als Feststoff, beispielsweise als Lyophilisat, vertrieben. Ein erfindungsgemässes Dosiergerät mit zwei oder mehr Behälterkammern erlaubt es, den Wirkstoff in einer ersten Kammer und das Lösungsmittel in einer zweiten Kammer bereitzustellen. Bevorzugt ist die Fördervorrichtung dabei derart fluidmässig zwischen der Kammer mit dem Wirkstoff, der Kammer mit dem Lösungsmittel und der Abgabeöffnung angeordnet, dass das Lösungsmittel in die Kammer mit dem Wirkstoff förderbar ist. Damit kann ein Auflösen des Wirkstoffes unmittelbar vor der Abgabe der Formulierung an einen Patienten erzielt werden. Dieselbe Fördervorrichtung ist darauf zur Förderung der abgabebereiten Lösung des Wirkstoffes zur der Abgabeöffnung einsetzbar.

Eine "fluidmässige Anordnung" bezeichnet hierbei eine Anordnung im Sinne eines fluidkommunizierenden Zusammenwirkens von Komponenten. Eine "fluidmässige Anordnung" eines Elementes zwischen zwei Komponenten weist somit wenigstens einen Fluidweg auf, welcher von einer Komponente zur anderen über das Element führt.

Zumindest ein Behälter kann zusätzlich ein Verschlussstück umfassen, wobei das Verschlussstück insbesondere fest im Behälter angeordnet und über einen Anschlusskanal mit der Fördervorrichtung in Fluidkommunikation bringbar ist. Ein solches Verschlussstück verhindert ein Kollabieren des Behälters beim Ansaugen des Fluids durch die Fördervorrichtung. Das Verschlussstück kann ferner dazu genutzt werden, um Teile der Fördervorrichtung in einen Behälter zu integrieren. Dabei müssen zum Beispiel keine oder nur wenige fluiddichte Verbindungen geschaffen werden, welche anfällig für Leckagen sein können. Bei geeigneter Ausbildung kann das Dosiergerät beispielsweise nur eine fluiddichte Verbindung erfordern, welche zum Beispiel die Auslassöffnung der Fördervorrichtung mit der Abgabeöffnung des Dosiergerätes verbindet.

Das Verschlussstück kann beispielsweise als einstückiges Spritzgussteil ausgebildet sein, in welchem Teile der Fördervorrichtung und/oder an diese anschliessende Fluidkanäle und Fluidöffnungen fest miteinander verbunden ausgebildet sein können. Auf diese Weise können fluidführende Komponenten in den Behälter integriert sein, womit zum Beispiel die Baugrösse des Dosiergerätes reduziert und/oder dessen Zuverlässigkeit erhöht werden kann.

Das Verschlussstück kann ferner eine mit dem Innenraum des Behälters kommunizierende Füllöffnung aufweisen, welche verschliessbar nach aussen offen ist. Zum luftfreien befüllen des Dosiergerätes kann das das gesamte fluidführende System durch eine solche Füllöffnung evakuiert werden.

Die Fördervorrichtung kann zusätzlich eine Analytiköffnung umfassen, welche in Längsrichtung zu der Ansaugöffnung oder den Ansaugöffnungen und der Auslassöffnung versetzt am Zylinder angeordnet und mit einer Analytikvorrichtung in Fluidverbindung bringbar ist. Dies ermöglicht neben der eigentlichen dosierten Abgabe eines Fluids, beispielsweise an einen Patienten, auch die Aufnahme von Flüssigkeiten zu deren Analyse. Das zu analysierende Fluid wird dabei durch die Analytiköffnung in oder durch eine Analytikvorrichtung gepumpt. Bei der Analytikvorrichtung kann es sich dabei beispielsweise um konventionelle Glukosemessstreifen oder um ein Spektrometer handeln. So ist es denkbar, dass bei einem Patienten, welcher unter Diabetes leidet, eine Probe am körpereigenen Fluid entnommen und analysiert wird, um die Dosierung einer Insulinformulierung zu bestimmen. Dadurch ist es möglich, den Blutzuckerspiegel des Patienten vollautomatisch zu regulieren.

Der erste Kolben kann von einem ersten Förderantrieb und der zweite Kolben von einem zweiten Förderantrieb antreibbar sein. Dies ermöglicht die beiden Kolben unabhängig voneinander anzutreiben, wodurch ein derartiges Dosiergerät für eine Vielzahl von Anwendungen geeignet ist.

Allerdings können der erste Kolben und der zweite Kolben auch von einem gemeinsamen Förderantrieb antreibbar sein. Zwar ist die Flexibilität einer solchen Konfiguration geringer als jene der oben beschriebenen, jedoch lässt sich ein derartiges Dosiergerät günstiger realisieren. So kann beispielsweise zumindest ein Kolben indirekt mit dem Förderantrieb in Wirkverbindung bringbar sein. Dadurch ist es möglich, dass die beiden Kolben von einem gemeinsamen Antrieb antreibbar sind und dennoch abweichende Hubbewegungen ausführen, wodurch das durch die beiden Stirnseiten der Kolben und der Zylinderinnenwand begrenzte Volumen über einen Arbeitszyklus der Fördervorrichtung variabel ist, was für eine Pumpwirkung unabdingbar ist.

In diesem Zusammenhang bezieht sich ein direkt mit einem Förderantrieb in Wirkverbindung stehender Kolben auf einen Kolben, der bei Bewegung des Förderantriebs stets eine Hubbewegung eingeht. Ein "indirekt" mit einem Förderantrieb in Wirkverbindung stehender Kolben bezieht sich dagegen auf einen Kolben, der bei Bewegung des Förderantriebs nur unter gewissen Umständen eine Hubbewegung eingeht.

Eine mögliche Ausführung eines solchen Dosiergerätes besteht darin, dass ein indirekt mit dem Förderantrieb in Wirkverbindung bringbarer Kolben über ein, vorzugsweise in seinem inneren angeordnetes, Federelement mit dem Förderantrieb in Wirkverbindung bringbar ist. Darüber hinaus können der erste Kolben und der zweite Kolben einstückig ausgebildet und über einen Faltenbalg miteinander verbunden sein. Diese Ausgestaltung ermöglicht es, die Fördervorrichtung in einer besonders einfachen Bauweise zu realisieren. Diese Bauform für die Fördervorrichtung ist auch als allgemeines Konstruktionsprinzip für Kolbenpumpen geeignet.

Zumindest ein Förderantrieb kann als Spindelantrieb ausgebildet sein. Ein Spindelantrieb hat den Vorteil, dass einerseits mit einem gängigen Elektromotor, welcher ein relativ geringes Drehmoment erzeugt, eine vergleichsweise hohe Kraft auf den Kolben ausübbar ist. Zudem lässt sich über eine geeignete Motorsteuerung, beispielsweise über eine Schrittmotorsteuerung, die Kolbenposition präzise einstellen.

Allerdings kann zumindest ein Förderantrieb auch ein Kurvengetriebe umfassen. Eine derartige Ausführung hat den Vorteil, dass durch eine geeignete Geometrie der Kurvenscheibe so gut wie jede beliebige Kolbenbewegung erzielbar ist. Ferner lässt sich ein Kurvengetriebe sehr preisgünstig realisieren und erlaubt eine im Vergleich zum Spindelantrieb deutlich schnellere Kolbenbewegung. Es versteht sich, dass der oder die Förderantriebe, bzw. die Kolben selbst, von einer im Dosiergerät angeordneten Überwachungsvorrichtung überwacht werden können. Auf diese Weise kann sichergestellt werden, dass eine aktuelle Kolbenstellung einer erwarteten Stellung entspricht.

Der oder die Förderantriebe können an die Fördervorrichtung koppelbar ausgelegt sein, so dass eine Antriebseinheit von einer Abgabeeinheit des Dosiergerätes trennbar ausgebildet sein kann. Dies kann zum Beispiel bei tragbaren Insulinabgabesystemen vorteilhaft sein, bei welchem die Antriebseinheit als wiederverwendbares Modul an jeweils unbenutzte Abgabeeinheiten abkoppelbar sein kann. Die Antriebseinheit umfasst bevorzugt einen Energiespeicher für den Förderantrieb sowie eine Steuereinheit zur Steuerung des Dosiergerätes. Ebenso können eine Analytikvorrichtung und/oder Kommunikationsmittel in der Antriebseinheit vorgesehen sein.

Ein erfindungsgemässes Dosiergerät kann eine Injektionsvorrichtung zur vorzugsweise kontinuierlichen subkutanen Abgabe eines Fluids an einen Patienten umfassen. Dadurch ist es möglich, beispielsweise bei einem Diabetiker ein erfindungsgemässes Dosiergerät an der Körperoberfläche anzubringen, um selbstständig über einen längeren Zeitraum ein medizinisches Fluid, beispielsweise eine Insulinlösung, an den Patienten abzugeben.

In einem ersten Aspekt der Erfindung umfasst das Dosiergerät ein Antriebsmodul und ein Abgabemodul, welche durch einen Benutzer verbindbar und voneinander trennbar ausgebildet sind. Das Antriebsmodul umfasst dabei zumindest Teile des Förderantriebs, insbesondere gegebenenfalls einen Drehantrieb und/oder gegebenenfalls einen Setzantrieb einer Injektionsvorrichtung. Das Abgabemodul weist zumindest den Behälter sowie die Fördervorrichtung und gegebenenfalls die Injektionsvorrichtung auf.

Ein weiterer Aspekt der vorliegenden Offenbarung betrifft ein Dosiergerät zur Verwendung mit einer Abfüllanlage zum Abfüllen eines Fluids unter aseptischen Bedingungen geeignet. Bei einem solchen Dosiergerät sind der erste Kolben und der zweite Kolben mit je einer Kolbenstange verbunden. Dabei ist zumindest eine Kolbenstange derart gekrümmt, dass ein Abschnitt gegensinnig parallel zu ihrem Kolben verläuft. Ferner kann die Fördervorrichtung ein Gehäuse umfassen, welches, vorzugsweise einstückig, mit dem Zylinder verbunden ist und einen Freiraum für den gekrümmten Bereich einer Kolbenstange sowie eine Parallelführung für den parallel zu ihrem Kolben verlaufenden Bereich aufweist. Das Gehäuse kann ein Auffangvolumen zum Auffangen von Fluid aufweisen. Ein solches Auffangvolumen kann bei bestimmungsgemässem Betrieb mit vertikaler Orientierung der Fördervorrichtung an den Kolben vorbeigeströmtes Fluid aussangen und durch eine Ablassöffnung in regelmässigen Zeitintervallen entleert werden. Die Ablassöffnung kann entsprechend verschliessbar ausgeführt sein.

Im Hinblick auf die erforderlichen Fertigungstoleranzen und die angestrebte Lebensdauer ist eine derartige Fördervorrichtung mit Vorteil aus Metall, insbesondere aus einem rostfreien Edelstahl, gefertigt. Die metallischen Teile können zusätzlich nichtmetallische Beschichtungen aufweisen. Es versteht sich von selbst, dass ein solches Dosiergerät ebenfalls mit Sensoren wie Druck- oder Temperatursensoren ausgestattet sein kann.

Ein derartiges Dosiergerät ist durch seine einfache Konstruktionsweise ohne Ventile und ohne Dichtungen oder Kolbenringe besonders gut zum Einsatz in einer Abfüllanlage in der pharmazeutischen Produktion geeignet, da es sich leicht demontieren, reinigen und sterilisieren lässt. Allerdings ist auch ein sog. cleaning in place (CIP) denkbar, wobei mit zusätzlichen Anschlüssen am Zylinder eine Hinterkolbenspülung durchführbar ist.

Weitere Vorteile und Einzelmerkmale der Erfindung ergeben sich aus der nachfolgenden Beschreibung mehrerer Ausführungsbeispiele und aus den Zeichnungen.

Es zeigen schematisch:
- Figur 1:: Eine perspektivische Darstellung einer erfindungsgemässen Dosiergerätes ;
- Figuren 2a-2f:: Eine Serie von Längsschnitten durch eine Fördervorrichtung eines erfindungsgemässen Dosiergerätes zur Veranschaulichung eines Pumpvorganges;
- Figur 3:: Ein Längsschnitt durch eine Fördervorrichtung eines erfindungsgemässen Dosiergerätes mit Drucksensor;
- Figuren 4a-4d:: Eine Serie von Längsschnitten durch eine Fördervorrichtung eines erfindungsgemässen Dosiergerätes zur Veranschaulichung eines Füllvorganges;
- Figuren 5a-5d:: Ein Längsschnitt durch eine Fördervorrichtung eines alternativen Ausführungsbeispiels eines erfindungsgemässen Dosiergerätes zur Veranschaulichung eines Füllvorganges;
- Figur 6:: Ein Längsschnitt durch eine Fördervorrichtung eines erfindungsgemässen Dosiergerätes mit zwei unabhängigen Förderantrieben;
- Figur 7:: Ein Längsschnitt durch eine Fördervorrichtung eines alternativen Ausführungsbeispiels einer erfindungsgemässen Dosiergerätes mit zwei unabhängigen Förderantrieben;
- Figur 8:: Ein Längsschnitt durch eine Fördervorrichtung eines erfindungsgemässen Dosiergerätes mit drei Ansaugöffnungen;
- Figuren 8a-8i:: Eine Serie von Teilvergrösserungen durch Längsschnitte einer Fördervorrichtung gemäss Figur 8 zur Veranschaulichung eines Pumpvorganges;
- Figur 9:: Ein Längsschnitt durch eine Fördervorrichtung eines erfindungsgemässen Dosiergerätes mit zusätzlicher Analytiköffnung;
- Figuren 9a-9j:: Eine Serie von Teilvergrösserungen von Längsschnitten durch eine Fördervorrichtung gemäss Figur 9 zur Veranschaulichung eines Analytikund Pumpvorganges;
- Figuren 10a-10e:: Eine Serie von Längsschnitten durch eine Fördervorrichtung eines erfindungsgemässen Dosiergerätes mit einem gemeinsamen Antrieb für beide Kolben;
- Figuren 11a-11e:: Eine Serie von Längsschnitten durch eine Fördervorrichtung eines alternativen Ausführungsbeispiels eines erfindungsgemässen Dosiergerätes mit einem gemeinsamen Antrieb für beide Kolben;
- Figur 12a:: Eine räumliche Darstellung eines Längsschnitts gemäss Figur 11c;
- Figur 12b:: Den durch einen Kreis markierten Faltenbalg 34 in Figur 12a, jedoch in ungeschnittener Form.
- Figuren 13a-13d:: Eine Serie von Längsschnitten durch eine Fördervorrichtung
mit zwei unabhängigen Kolbenantrieben, welche als Kurvengetriebe ausgebildet sind;
- Figuren 14a-14e:: Eine Serie von Längsschnitten durch eine Fördervorrichtung zum Einbau in eine Abfüllanlage für flüssige Pharmazeutika.

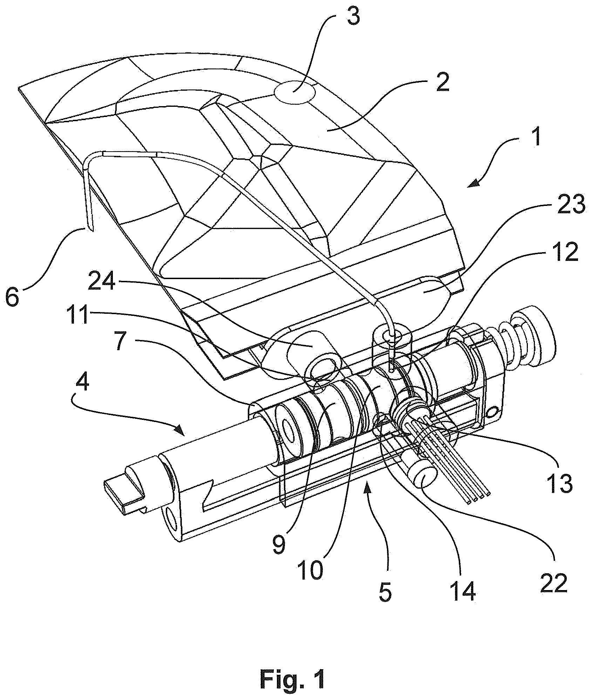

Figur 1 zeigt eine perspektivische Darstellung eines erfindungsgemässen Dosiergerätes 1. Das besagte Dosiergerät umfasst zusätzlich einen kollabierbaren Behälter 2, in dessen Innenraum 3 das abzugebende Fluid enthalten ist. Der Behälter 2 ist über einen Anschlusskanal 24 im Verschlussstück 23 mit der Fördervorrichtung 5 verbunden. Die Fördervorrichtung 5 umfasst einen Zylinder 7, in welchem ein erster Kolben 9 und ein zweiter Kolben 10 verschiebbar gelagert sind. Am Zylinder 7 ist eine Ansaugöffnung 11 zum Ansaugen des Fluids aus dem Behälter 2 angebracht.

Zur Abgabe des Fluids vom Dosiergerät 1 ist am Zylinder 7 zudem eine Auslassöffnung 12 angebracht, die fluidmässig mit der Abgabeöffnung 6 verbunden ist. Zusätzlich sind im gezeigten Ausführungsbeispiel ein Drucksensor 13 sowie eine Füllöffnung 14, welche mit einem Stopfen 22 verschlossen ist, am Zylinder 7 angebracht. Der erste Kolben 9 und der zweite Kolben 10 werden im gezeigten Ausführungsbeispiel durch einen gemeinsamen Förderantrieb 4, welcher hier als Spindelantrieb ausgebildet ist, angetrieben.

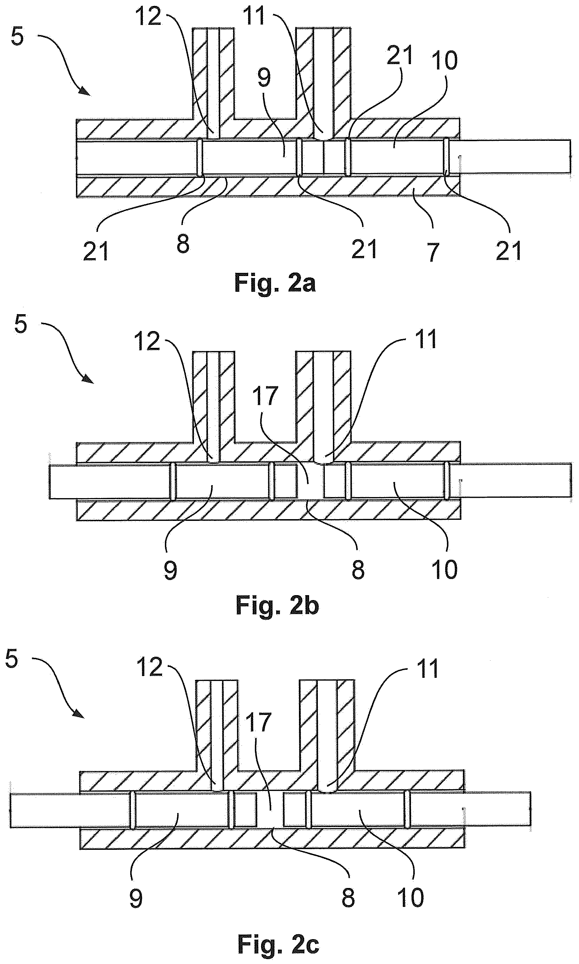

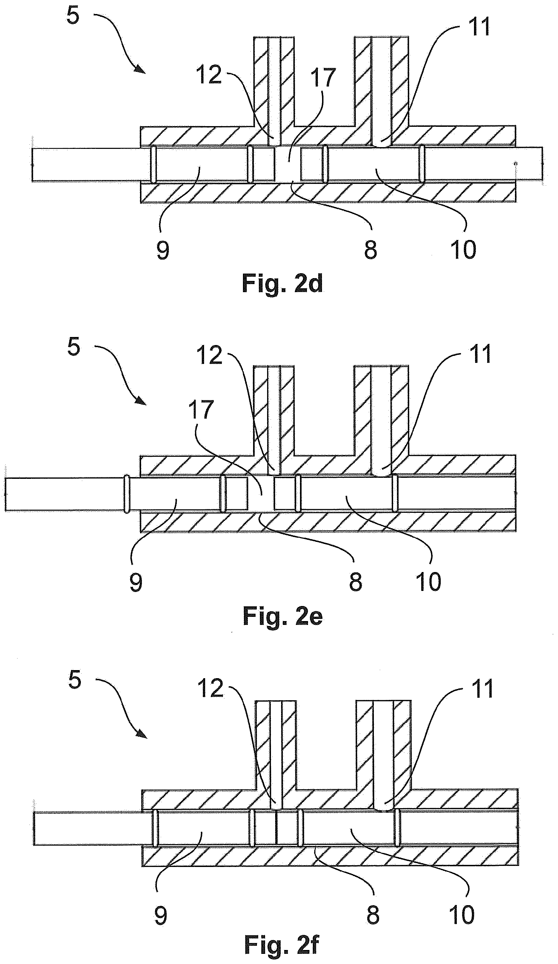

In den Figuren 2a-f ist das Funktionsprinzip der Fördervorrichtung 5 eines Dosiergerätes 1 gemäss Figur 1 dargestellt. Figur 2a zeigt einen Längsschnitt durch die Fördervorrichtung 5 in ihrem Grundzustand. Die beiden Kolben 9 und 10 sind im Zylinder 7 verschiebbar gelagert. Um einen fluiddichten Kontakt zwischen den Kolben 9 und 10 und der Zylinderinnenwand 8 herzustellen, sind an den Kolben 9 und 10 mehrere Dichtungsringe 21 angebracht. Am Zylinder 7 der Fördervorrichtung 5 sind zudem eine Ansaugöffnung 11 und eine Auslassöffnung 12 vorhanden. In der Grundstellung der Fördervorrichtung 5 sind die beiden Kolben 9 und 10 mit stirnseitigem Kontakt auf Höhe der Ansaugöffnung 11 positioniert. Figur 2b zeigt die Fördervorrichtung 5 nach erfolgtem Ansaugen eines Fluids. Es ist zu erkennen, dass der erste Kolben 9 in Richtung der Auslassöffnung 12 verschoben wurde, wodurch sich zwischen den Stirnseiten der beiden Kolben 9 und 10 ein von innen und der Zylinderinnenwand 8 begrenztes Volumen 17 gebildet hat. In Figur 2c sind die beiden Kolben 9 und 10 in gleichem Abstand zueinander in Richtung der Auslassöffnung 12 verschoben. In Figur 2d hat der erste Kolben 9 in Längsrichtung des Zylinders 7 die Höhe der Auslassöffnung 12 erreicht. Von dieser Position aus kann der zweite Kolben 10 zum Ausstossen der Flüssigkeit in Richtung des ersten Kolbens 9 weiter verschoben werden, bis die beiden Kolben 9 und 10 wie in Figur 2f gezeigt stirnseitigen Kontakt haben. Allerdings können die beiden Kolben 9 und 10 ausgehend von einer Stellung gemäss Figur 2d auch in gleichem Abstand weiterverschoben werden, bis sich der zweite Kolben 10 auf Höhe der Auslassöffnung 12 befindet, was der Figur 2e entspricht. Von hier aus ist ein alternatives Ausstossprinzip realisierbar, bei welchem der erste Kolben 9 in Richtung des zweiten Kolbens 10 verschoben wird.

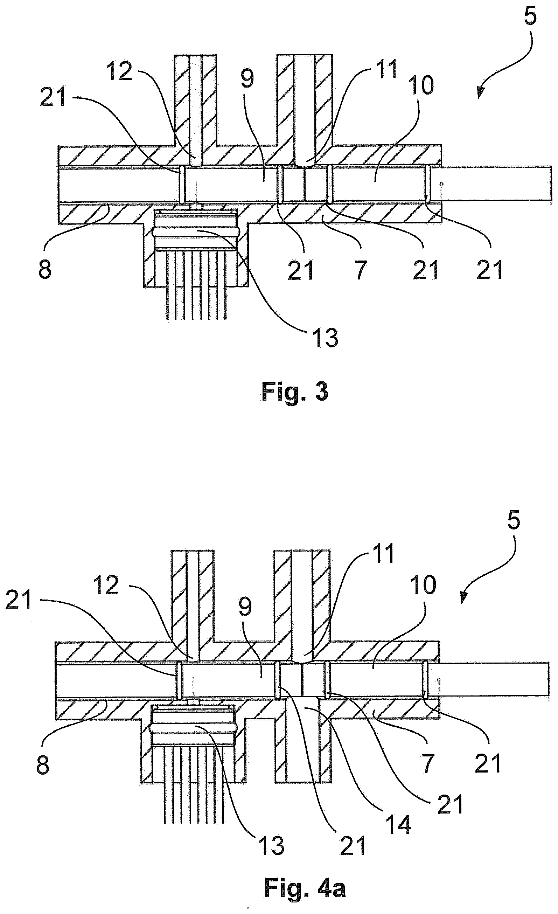

Figur 3 zeigt ein alternatives Ausführungsbeispiel einer Fördervorrichtung 5 eines erfindungsgemässen Dosiergerätes 1, bei welcher zusätzlich auf Höhe der Auslassöffnung 12 am Zylinder 7 ein Drucksensor 13 angebracht ist.

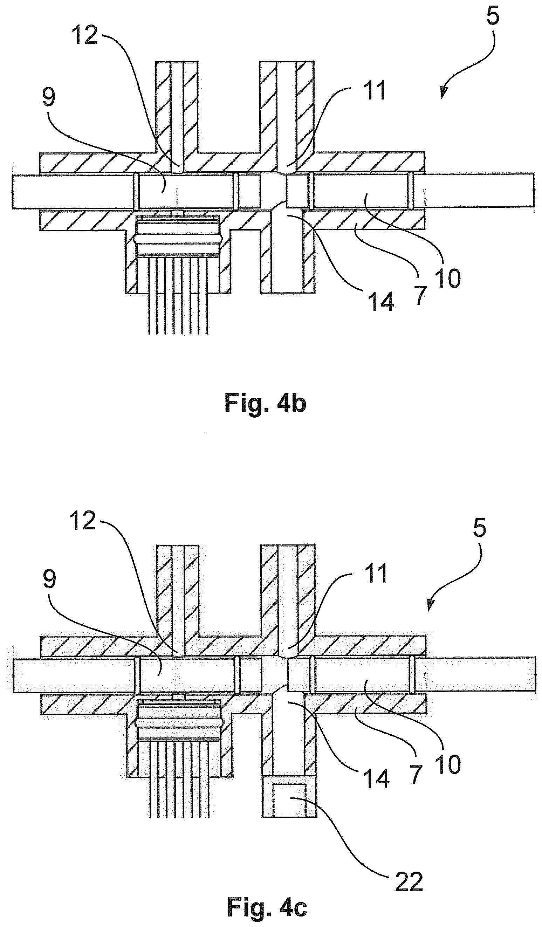

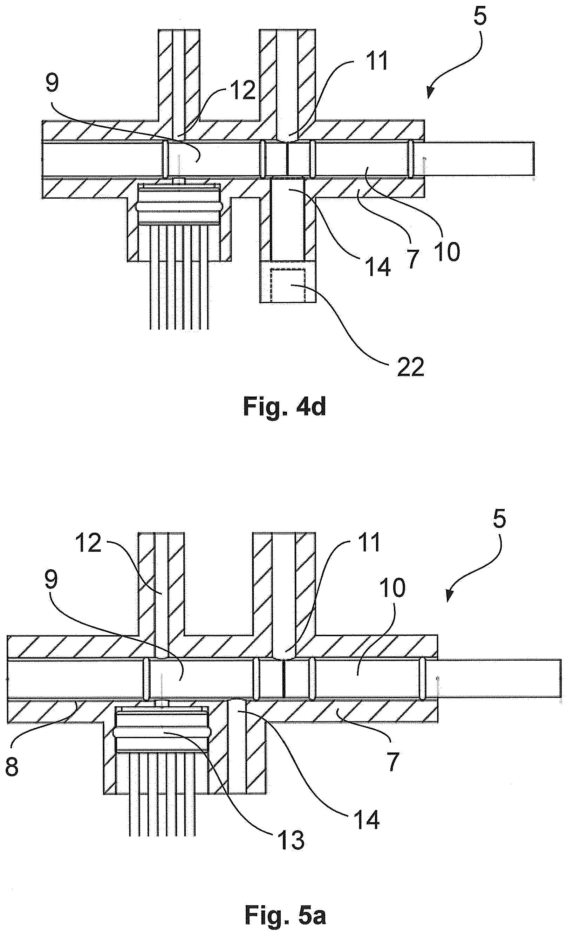

In den Figuren 4a-d ist eine Sequenz von Längsschnitten durch die Fördervorrichtung 5 eines weiteren Ausführungsbeispiels eines erfindungsgemässen Dosiergerätes 1 gezeigt. Bei der besagten Fördervorrichtung ist am Zylinder 7 eine zusätzliche Füllöffnung 14 angebracht. Figur 4a zeigt die Fördervorrichtung 5 in ihrer Grundposition vor Befüllen des Behälters 2. Die beiden Kolben 9 und 10 sind mit stirnseitigem Kontakt in Längsrichtung des Zylinders 7 auf Höhe der Ansaugöffnung 11 und der Füllöffnung 14 positioniert. Wie in Figur 4b zu erkennen ist, wird durch Verschieben des ersten Kolbens 9 in Richtung der Auslassöffnung 12 eine Fluidverbindung zwischen der Füllöffnung 14 und der Ansaugöffnung 11 hergestellt. Der Innenraum 3 des Behälters 2 kann durch diese Fluidverbindung mit Fluid befüllt werden. Nach erfolgtem Füllvorgang wird die Füllöffnung 14 gemäss Figur 4c mit einem Stopfen 22 verschlossen. In Figur 4d ist die Fördervorrichtung 5 mit gefüllten Behälter 2 erneut in ihrer Grundposition dargestellt.

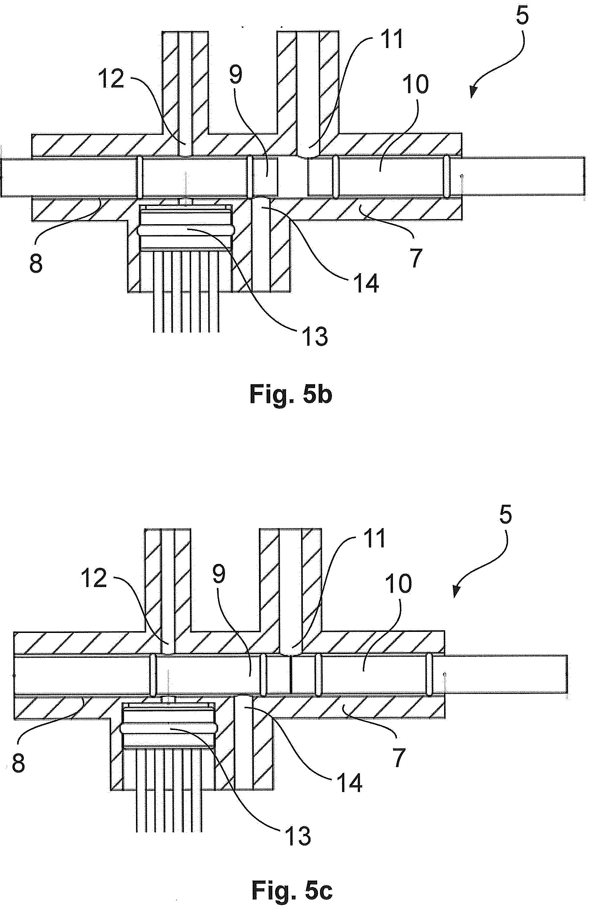

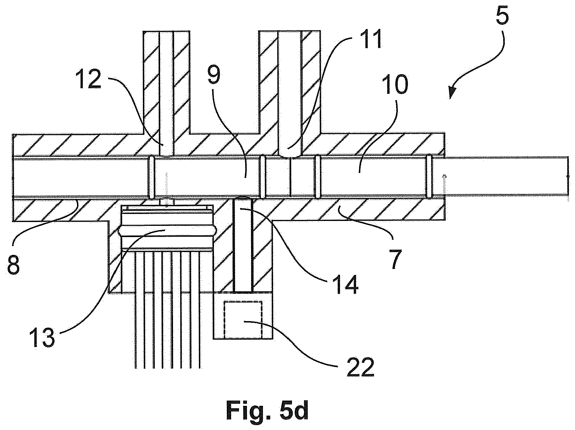

Die Figuren 5a-d verdeutlichen den Füllvorgang eines alternativen Ausführungsbeispiels eines erfindungsgemässen Dosiergerätes 1. In Figur 5a ist ein Längsschnitt durch eine entsprechende Fördervorrichtung 5 gezeigt, bei welcher die Füllöffnung 14 und die Ansaugöffnung 11 in Längsrichtung versetzt am Zylinder 7 angeordnet sind. Zum Befüllen des Dosiergerätes 1 wird der erste Kolben 9 in Richtung der Auslassöffnung 12 verschoben (Figur 5b), um eine Fluidverbindung zwischen der Füllöffnung 14 und der Ansaugöffnung 11 herzustellen. Wie in Figur 5c dargestellt, kann die besagte Fluidverbindung nach erfolgtem Befüllen des Behälters 2 durch erneutes Verschieben des ersten Kolbens 9 in Richtung des zweiten Kolbens 10 wieder unterbrochen werden. Als letzter Schritt des Füllvorgangs wird die Füllöffnung 14 durch einen Stopfen 22 verschlossen (Figur 5d).

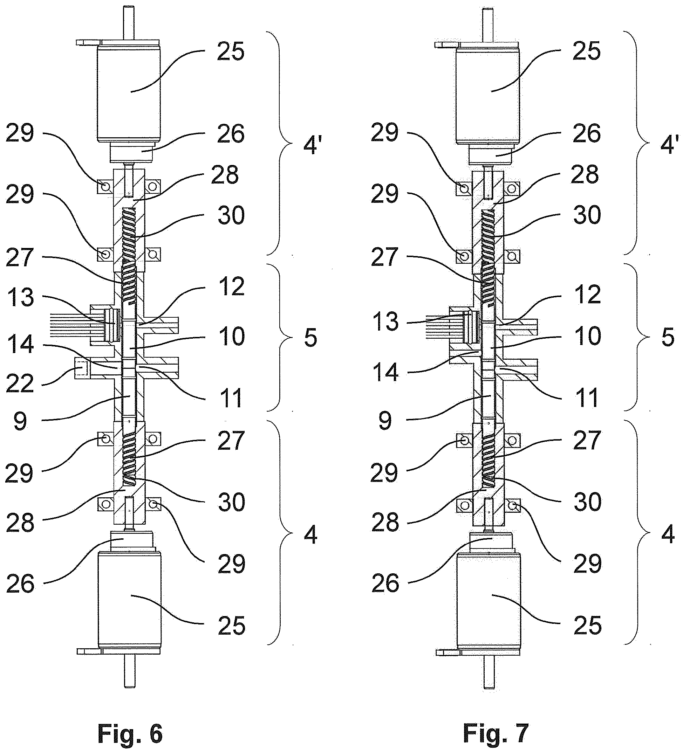

Die Figur 6 zeigt einen Teilschnitt durch ein erfindungsgemässes Dosiergerät 1, bei welchem die Fördervorrichtung 5 durch zwei unabhängige Förderantriebe 4 und 4' angetrieben wird. Die beiden Förderantriebe 4 und 4' sind dabei identisch aufgebaut und umfassen je einen Motor 25 mit einem Getriebe 26. Die mit dem Motor 25 erzeugte Drehbewegung wird auf eine Spindelhülse 28 übertragen, welche durch die Lager 29 drehbar gelagert ist. Die Spindelhülse 28 weist eine Bohrung mit einem Gewinde 30 auf, in welches die Spindel 27 greift. Dadurch kann eine von Motor 25 und Getriebe 26 erzeugte Drehbewegung in eine Längsbewegung des Kolbens 9 oder 10 umgewandelt werden. Im besagten Ausführungsbeispiel ist an der Fördervorrichtung 5 eine Füllöffnung 14 in Längsrichtung auf gleicher Höhe mit der Ansaugöffnung 11 angebracht und über einen Stopfen 22 verschlossen.

Die Figur 7 zeigt ein alternatives Ausführungsbeispiel eines erfindungsgemässen Dosiergerätes 1 mit den Förderantrieben 4 und 4' gemäss Figur 6. Allerdings ist hier am Zylinder 7 der Fördervorrichtung 5 die Füllöffnung 14 in Längsrichtung versetzt zur Ansaugöffnung 11 angeordnet.

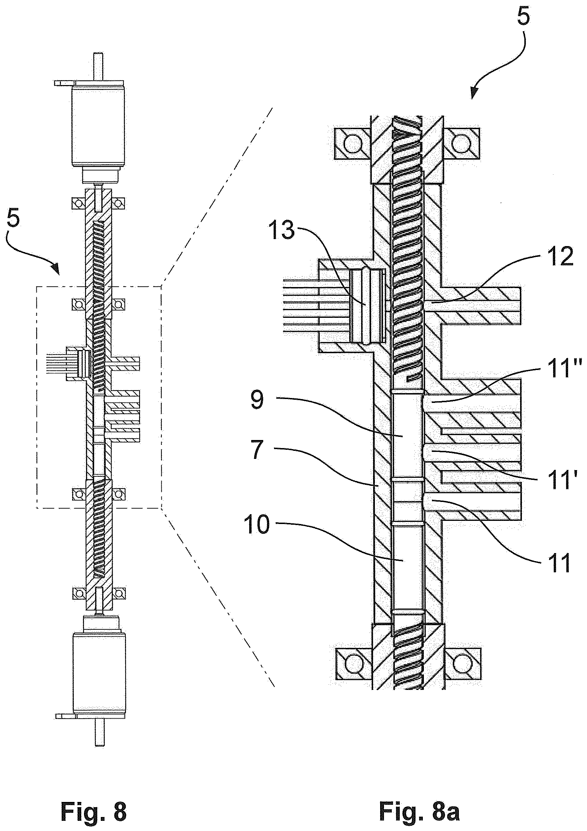

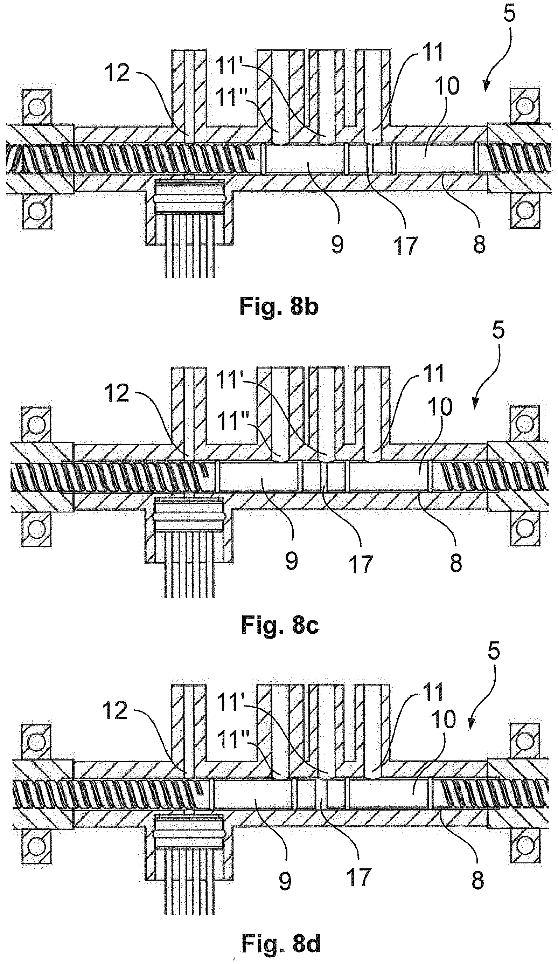

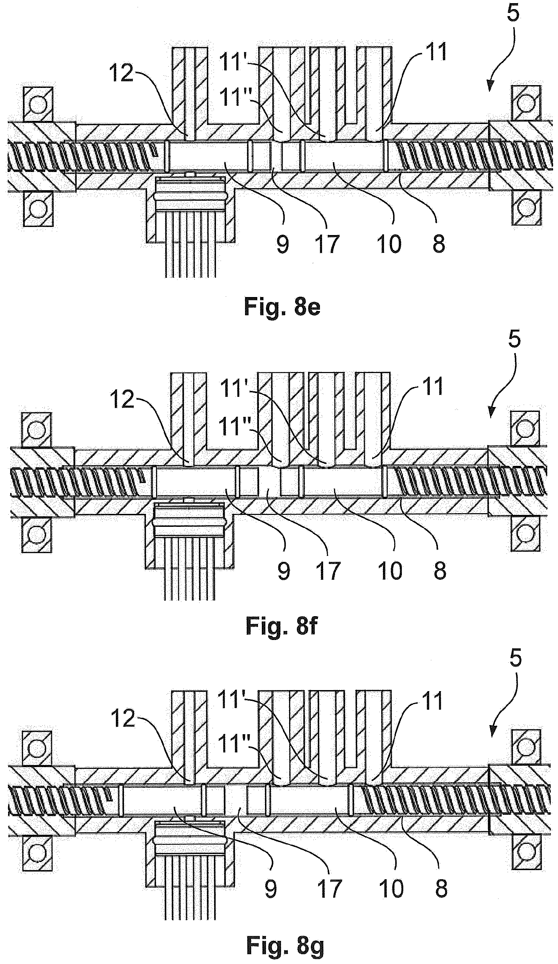

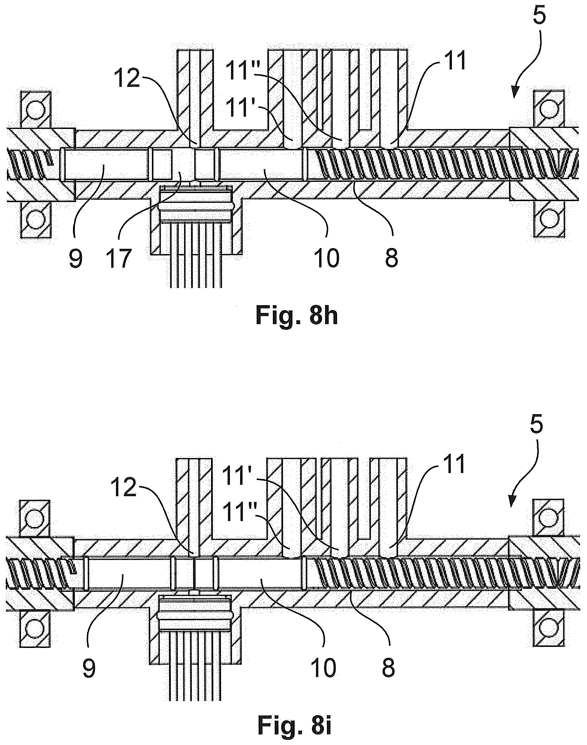

Die Figur 8 zeigt ein weiteres Ausführungsbeispiel eines erfindungsgemässen Dosiergerätes 1 mit einer Fördervorrichtung 5, welche drei unabhängige Ansaugöffnungen 11, 11' und 11" aufweist. Die Figuren 8a-i stellen eine Serie von Längsschnitten der besagten Fördervorrichtung 5 dar, welche einer Teilvergrösserung von Figur 8 entsprechen. In Figur 8a ist die Fördervorrichtung 5 in ihrer Grundposition gezeigt, wobei sich der erste Kolben 9 und der zweite Kolben 10 auf Höhe der ersten Ansaugöffnung 11 stirnseitig berühren. In Figur 8b ist der erste Kolben 9 in Richtung der Auslassöffnung 12 verschoben, wodurch zwischen den Stirnseiten des ersten Kolbens 9 und des zweiten Kolbens 10 und der Zylinderinnenwand 8 ein Volumen 17 zum Ansaugen des Fluids gebildet wird. In Figur 8c sind die beiden Kolben 9 und 10 in gleichem Abstand in Zylinderlängsrichtung auf Höhe der zweiten Ansaugöffnung 11' verschoben. Durch erneutes Bewegen des ersten Kolbens 9 in Richtung der Auslassöffnung 12 kann ein weiteres Fluid durch die Ansaugöffnung 11' angesogen werden (Figur 8d). In Figur 8e sind die beiden Kolben 9 und 10 erneut in gleichem Abstand in Richtung der Abgabeöffnung 12 auf Höhe der dritten Ansaugöffnung 11" versetzt. Auch hier kann durch Verschieben des ersten Kolbens 9 in Richtung der Auslassöffnung 12 Fluid durch die Ansaugöffnung 11" angesaugt werden (Figur 8f). Figur 8g zeigt das äquidistante Verschieben des ersten Kolbens 9 und des zweiten Kolbens 10 zu der Abgabeöffnung 12. Figur 8h stellt die Fördervorrichtung 5 in abgabebereitem Zustand mit dem zweiten Kolben 10 auf Höhe der Auslassöffnung 12 dar. Nach Abgabe des Fluids durch Verschieben des ersten Kolbens 9 in Richtung des zweiten Kolbens 10 besteht, wie in Figur 8i gezeigt, wieder stirnseitiger Kontakt zwischen den beiden Kolben 9 und 10.

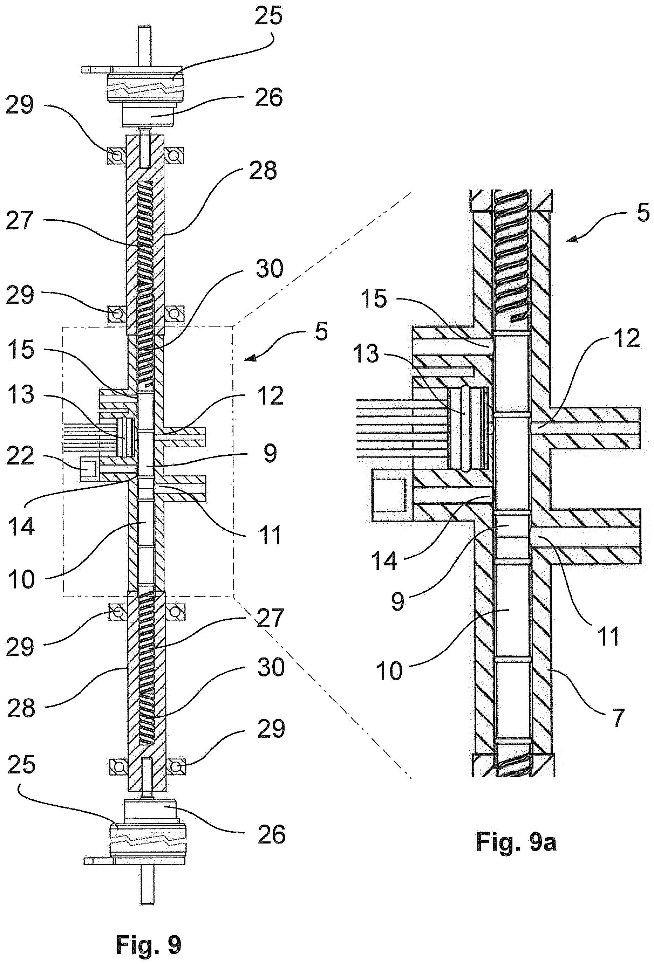

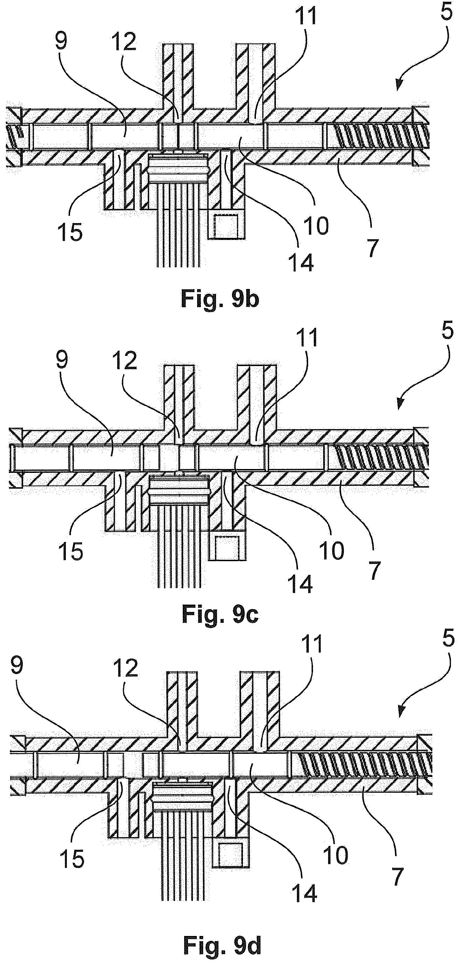

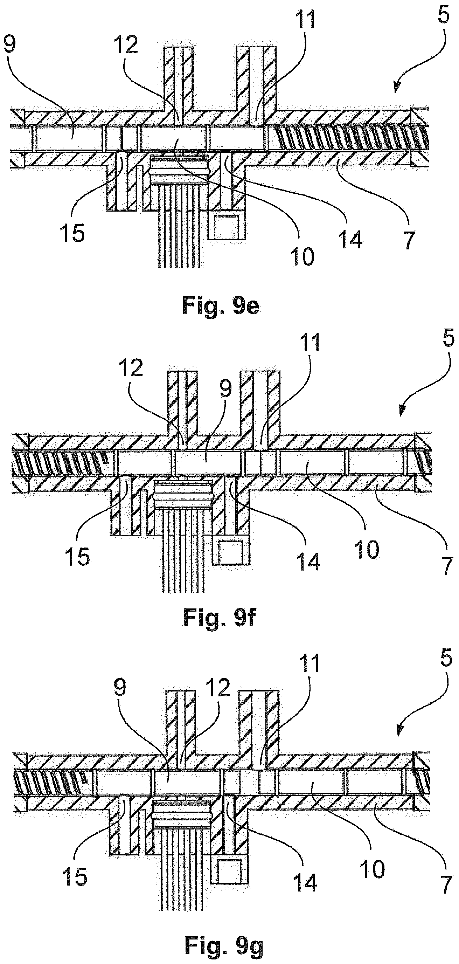

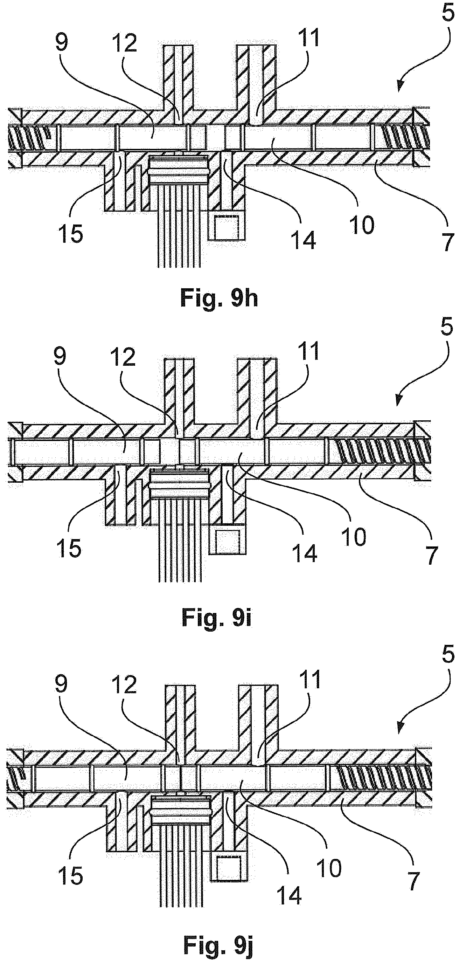

Die Figur 9 zeigt einen Längsschnitt durch ein weiteres Ausführungsbeispiel eines erfindungsgemässen Dosiergerätes 1 mit einer zusätzlichen Analytiköffnung 15 am Zylinder 7 der Fördervorrichtung 5. In Figur 9a ist die entsprechende Fördervorrichtung 5 in Teilvergrösserung gezeigt. Die beiden Kolben 9 und 10 befinden sich in der Grundposition mit ihren Stirnflächen in Längsrichtung des Zylinders 7 auf Höhe der Ansaugöffnung 11. In einem ersten Schritt werden die beiden Kolben 9 und 10 auf Höhe der Auslassöffnung 12 verschoben, wie es in Figur 9b verdeutlicht ist. Darauf wird durch Bewegen des ersten Kolbens 9 in Richtung des Analytiköffnung 15 durch die Auslassöffnung 12 ein Fluid, beispielsweise Blut von einem Patienten, in das Dosiergerät 1 aufgenommen (Figur 9c). Das aufgenommene Fluid wird darauf durch äquidistantes Verschieben der Kolben 9 und 10 zur Analytiköffnung 15 gebracht (Figur 9d). Durch Bewegen des zweiten Kolbens 10 in Richtung des ersten Kolbens 9 wird das Fluid durch die Analytiköffnung 15 an eine Analytikvorrichtung abgegeben. Nach erfolgter Abgabe liegt die Fördervorrichtung 5 mit stirnseitigem Kontakt des ersten Kolbens 9 und des zweiten Kolbens 10 gemäss Figur 9e vor. Die Kolben werden danach gemeinsam in die Grundposition gemäss 9f versetzt. Wie in den Figuren 9g-j gezeigt, ,kann darauf eine mit der Analytikvorrichtung ermittelte Dosis eines Fluids in die Fördervorrichtung 5 aufgezogen und an einen Patienten abgebeben werden.

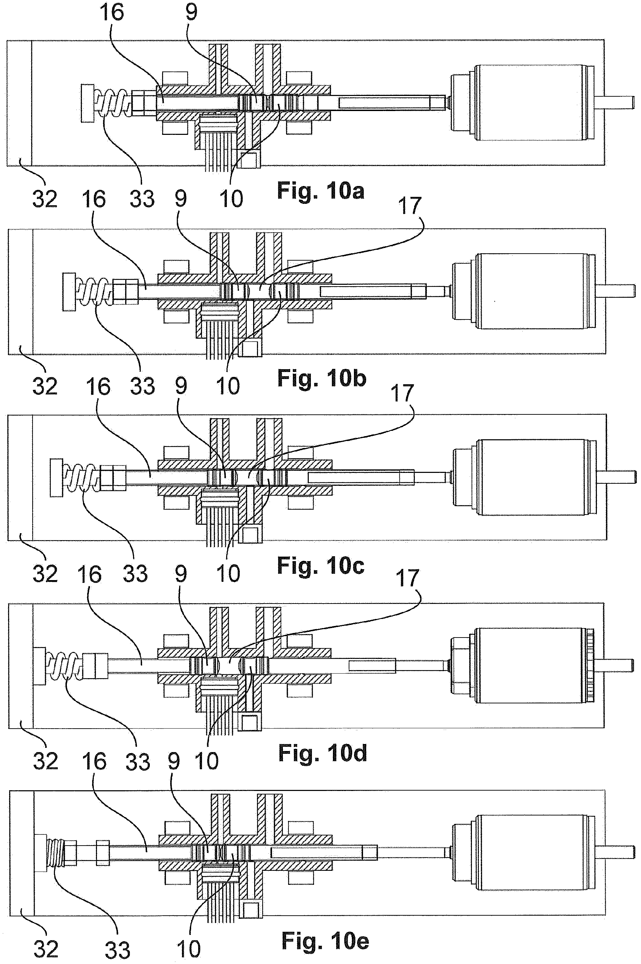

Die Figuren lOa-e zeigen ein Ausführungsbeispiel eines erfindungsgemässen Dosiergerätes 1, bei welchem die beiden Kolben 9 und 10 der Fördervorrichtung 5 durch einen gemeinsamen Förderantrieb 4 angetrieben werden. Der erste Kolben 9 ist dabei indirekt über eine Druckfeder 33 an den Förderantrieb 4 gekoppelt, während der zweite Kolben 10 schwimmend mit dem Förderantrieb 4 verbunden ist. Wie in Figur 10b zu erkennen ist, führt eine Betätigung des Förderantriebs ausgehend von der Grundposition in Figur 10a erst zum alleinigen Verschieben des ersten Kolbens 9 in Richtung der Auslassöffnung 12. Ist das gewünschte Fluidvolumen 17 zwischen den beiden Kolben 9 und 10 erreicht, wird wie in Figur 10c gezeigt der zweite Kolben 10 in gleichbleibendem Abstand zum ersten Kolben 9 in Richtung der Abgabeöffnung 12 verschoben. In Figur 10d hat der erste Kolben 9 in Längsrichtung des Zylinders 7 die Höhe der Abgabeöffnung 12 erreicht. Es ist zu erkennen, dass die Kolbenstange 16 des ersten Kolbens 9 an diesem Punkt auch den Anschlag 32 berührt. Eine weitere Betätigung des Förderantriebs 4 führt wie in Figur 10e gezeigt zum Komprimieren der Feder 33, wodurch der zweite Kolben 10 weiter in Richtung der Abgabeöffnung 12 verschoben wird, was wiederum zu einer Abgabe des aufgezogenen Fluidvolumens führt.

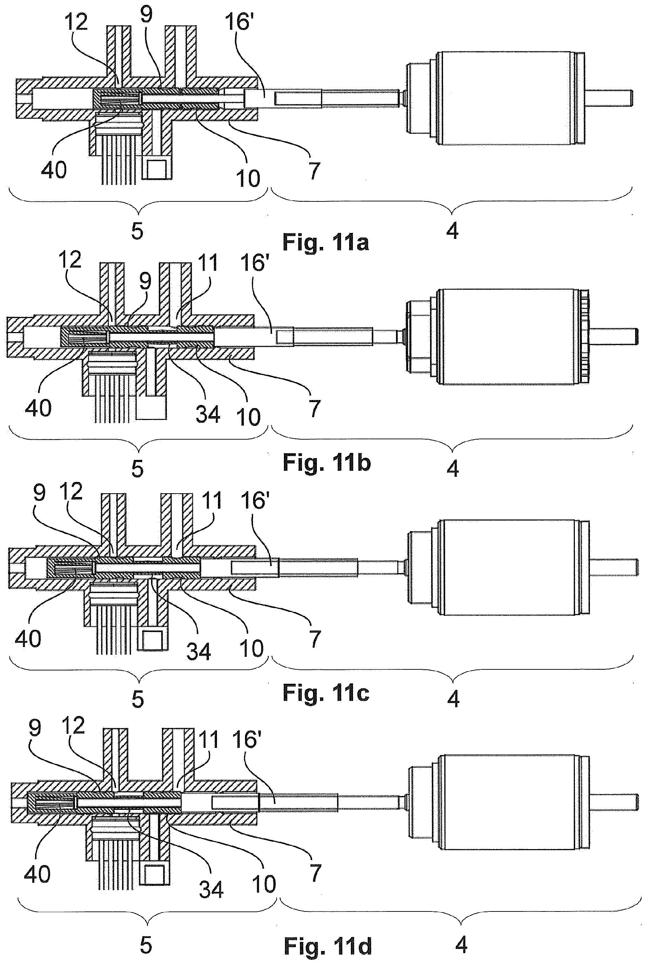

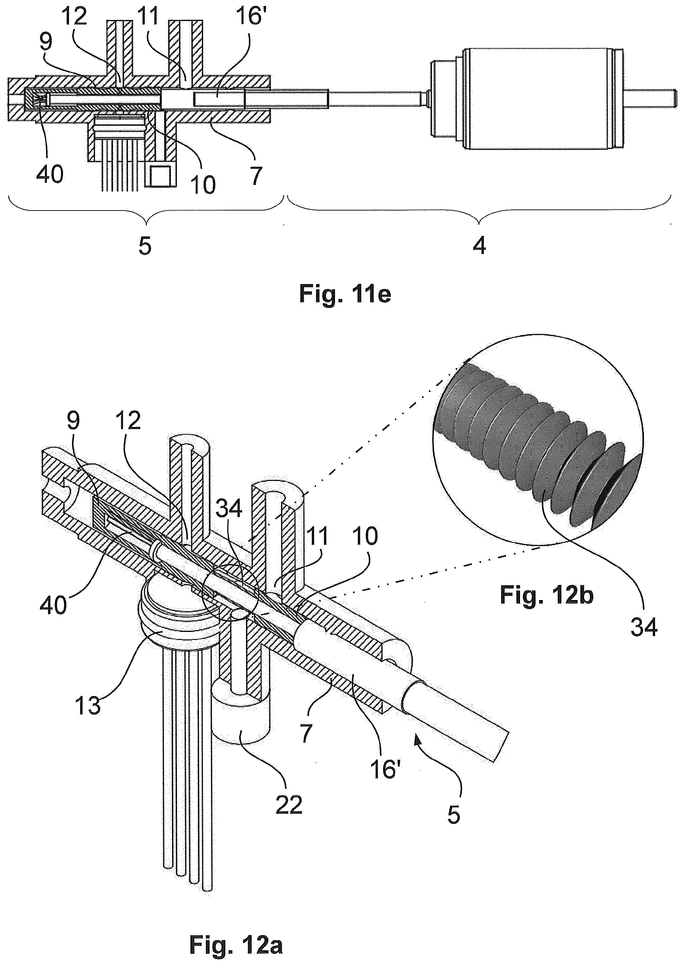

Die Figuren 11a-e zeigen ein weiteres Ausführungsbeispiel eines erfindungsgemässen Dosiergerätes 1, bei welchem die Fördervorrichtung 5 mit nur einem Förderantrieb 4 angetrieben wird. Der erste Kolben 9 steht dabei über ein in seinem inneren angeordnetes Federelement 40 mit dem Förderantrieb 4 indirekt in Wirkverbindung. Der zweite Kolben 10 kann durch die Kolbenstange 16' in Richtung der Auslassöffnung 12 und durch den ersten Kolben 9 in Richtung der Ansaugöffnung 11 geschoben werden. Der erste Kolben 9 und der zweite Kolben 10 sind einstückig ausgebildet und über einen Faltenbalg 34 miteinander verbunden. Auf diese Weise können die Kolben 9 und 10 sowie der Faltenbalg 34 aus einem einzigen Teil, beispielsweise aus Silikon, TPE oder Brombutylkautschuk, realisiert werden.

Die Figur 12a zeigt eine räumliche Darstellung eines Längsschnittes gemäss der Figur 11c. Es ist zu erkennen dass der erste Kolben 9 und der zweite Kolben 10 äquidistant in Richtung der Auslassöffnung 12 verschoben werden. Dabei wird der erste Kolben 9 über das Federelement 40 bewegt und der zweite Kolben 10 von der Kolbenstange 16' geschoben. Die Figur 12b zeigt den in der Figur 12a durch einen Kreis markierten Faltenbalg 34, jedoch nicht als Teilschnitt, sondern in räumlicher Darstellung.

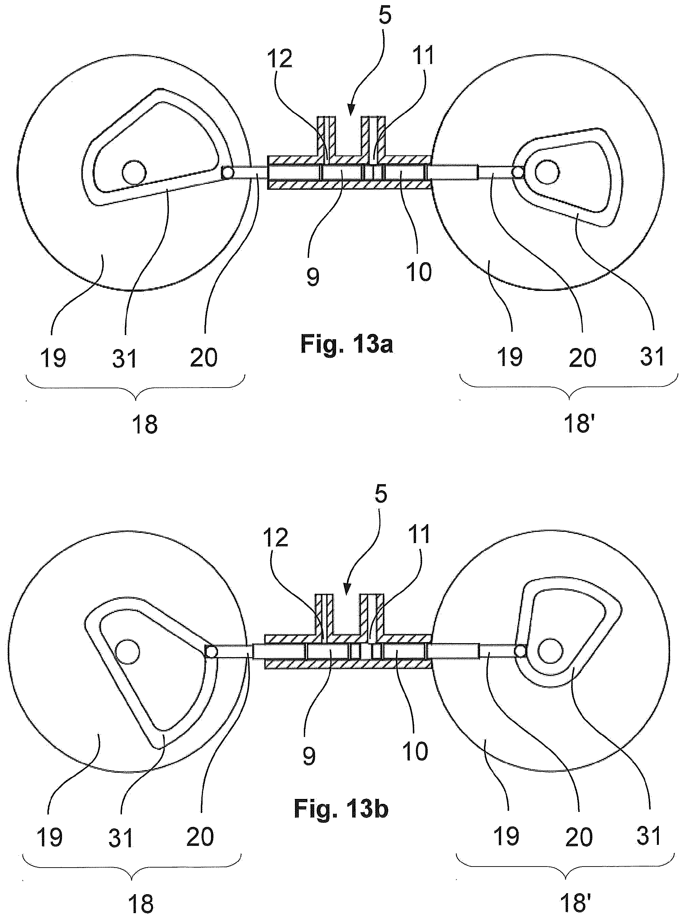

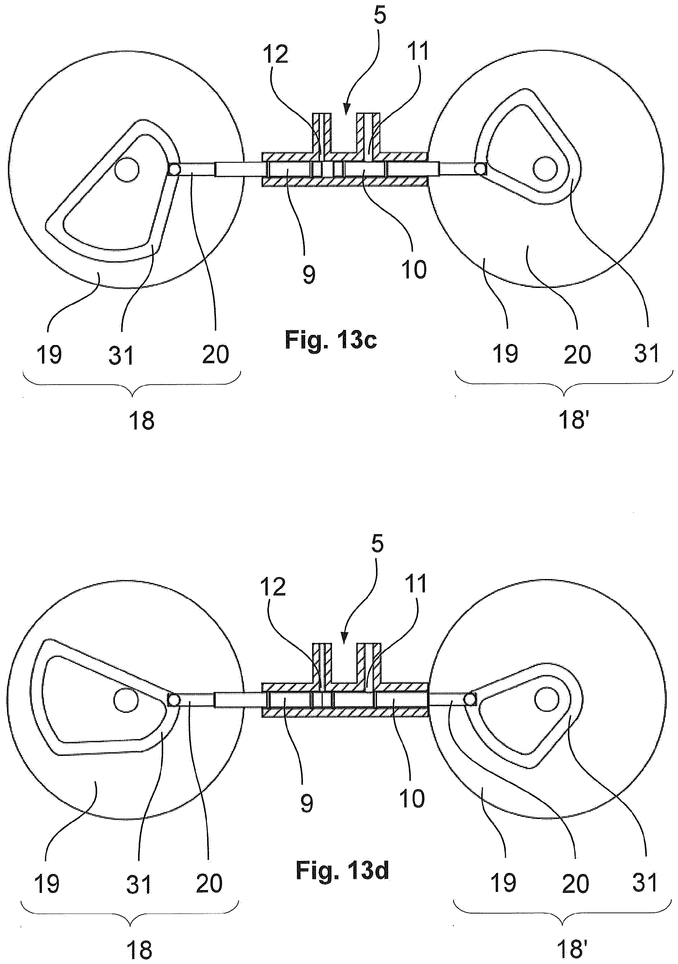

Die Figuren 13a-d zeigen ein weiteres Dosiergerät 1, bei welchem die Kolben 9 und 10 der Fördervorrichtung 5 durch zwei unabhängige Förderantriebe 4 und 4' angetrieben werden. Allerdings handelt es sich in diesem Fall um zwei Kurvengetriebe 18 und 18'. Die besagten Kurvengetriebe 18 und 18' umfassen jeweils eine Steuerscheibe 19 mit einer Führungsnut 31, über welche ein Stössel 20 angelenkt ist.

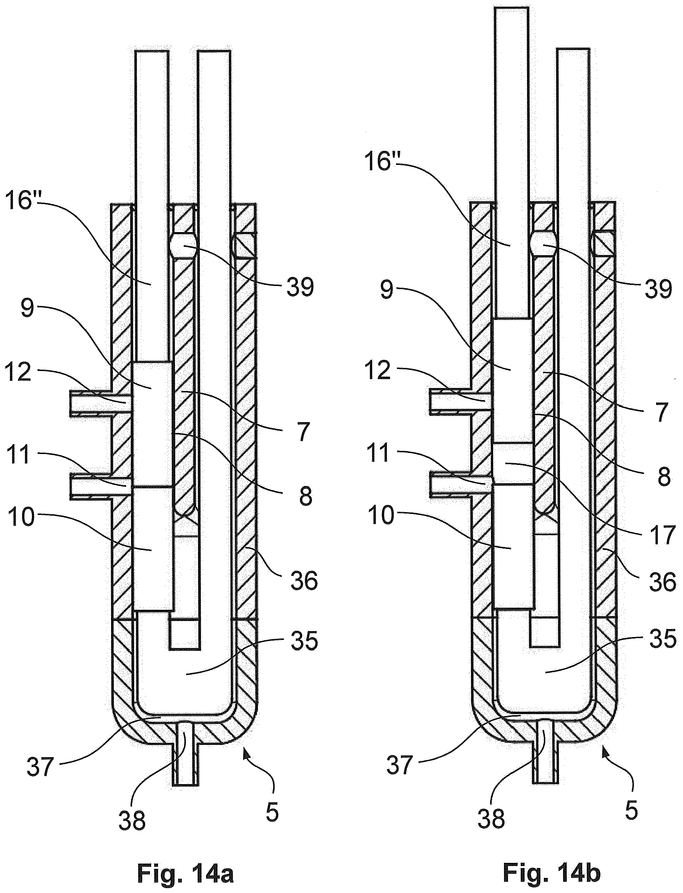

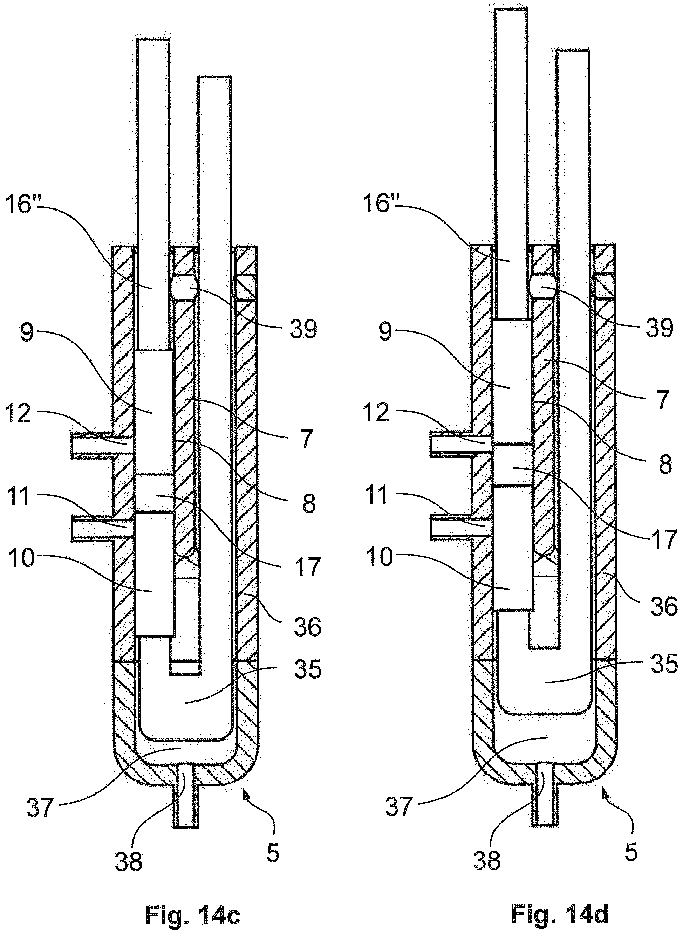

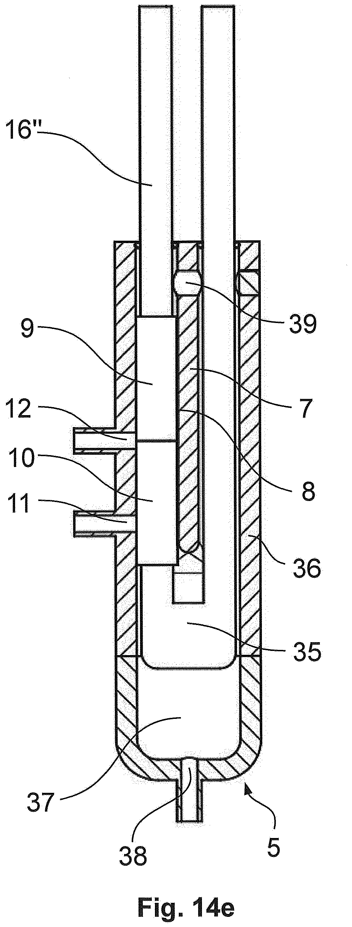

Die Figuren 14a-e zeigen die Fördervorrichtung 5 zum Einbau in eine Abfüllanlage für flüssige pharmazeutische Formulierungen. Die Fördervorrichtung 5 weist in diesem Fall einen Zylinder 7 auf, welcher einstückig als U-förmiges Gehäuse 36 ausgeführt ist. Während der erste Kolben 9 in eine reguläre gerade Kolbenstange 16" übergeht, ist der zweite Kolben 10 mit einer U-förmigen Kolbenstange 35 verbunden, welche eine Biegung um 180° aufweist. Die Fördervorrichtung 5 ist nicht nur ventillos, sondern auch ohne Dichtungen oder Kolbenringe an den Kolben konzipiert. Das Gehäuse 36 weist einen Raum 37 für die Biegung der Kolbenstange 35 sowie Kanäle 38 und 39 zum Spülen der Fördervorrichtung 5 auf.

## Patentansprüche

1. Dosiergerät (1) zur Abgabe eines Fluids unter aseptischen Bedingungen, umfassend zumindest einen Behälter (2) mit einem Innenraum (3) und eine von zumindest einem Förderantrieb (4) angetriebene Fördervorrichtung (5) zur Förderung des Fluids aus dem Innenraum (3) des zumindest einen Behälters (2), wobei das Fluid mittels der Fördervorrichtung (5) vom Behälter (2) zu einer Abgabeöffnung (6) förderbar ist, wobei die Fördervorrichtung (5) einen Zylinder (7) mit wenigstens einer Ansaugöffnung (11) und wenigstens einer Auslassöffnung (12) an einer Zylinderinnenwand (8), sowie einen ersten Kolben (9) und einen zweiten Kolben (10) umfasst, wobei der erste Kolben (9) und der zweite Kolben (10) innerhalb des Zylinders (7) in Längsrichtung verschiebbar gelagert sind und wobei der erste Kolben (9) und der zweite Kolben (10) zwischen ihren Stirnseiten gemeinsam mit einem Abschnitt der Zylinderinnenwand (8) ein variables Fluidvolumen (17) begrenzen, **dadurch gekennzeichnet, dass** das Dosiergerät (l) ein Antriebsmodul und ein Abgabemodul umfasst, welche durch einen Benutzer verbindbar und voneinander trennbar ausgebildet sind, wobei das Antriebsmodul zumindest Teile des Förderantriebs (4), insbesondere gegebenenfalls einen Drehantrieb, und/oder gegebenenfalls einen Setzantrieb einer Injektionsvorrichtung umfasst, und das Abgabemodul zumindest den Behälter (2) sowie die Fördervorrichtung (5) und gegebenenfalls die Injektionsvorrichtung aufweist, und der Behälter (2) zumindest teilweise kollabierbar ist.

2. Dosiergerät (1) gemäss Anspruch 1, **dadurch gekennzeichnet, dass** die Ansaugöffnung (11) und die Auslassöffnung (12) in Längsrichtung versetzt am Zylinder (7) angeordnet sind.

3. Dosiergerät (1) gemäss einem der Ansprüche 1 oder 2, **dadurch gekennzeichnet, dass** die Ansaugöffnung (11) mit dem Innenraum (3) eines Behälters (2) und die Auslassöffnung (12) mit der Abgabeöffnung (6) in Fluidverbindung bringbar sind.

4. Dosiergerät (1) gemäss einem der Ansprüche 1 bis 3, **dadurch gekennzeichnet, dass** die Fördervorrichtung (5) einen Drucksensor (13) aufweist, welcher vorzugsweise am Zylinder (7), insbesondere in Längsrichtung auf Höhe der Auslassöffnung (12), angeordnet ist.

5. Dosiergerät (1) gemäss einem der Ansprüche 1 bis 4, **dadurch gekennzeichnet, dass** die Fördervorrichtung (5) zusätzlich eine Füllöffnung (14) umfasst, welche durch verschieben des ersten Kolbens (9) und des zweiten Kolbens (10) innerhalb des Zylinders (7) mit der Ansaugöffnung (11) in Fluidkommunikation bringbar ist.

6. Dosiergerät (1) gemäss Anspruch 5, **dadurch gekennzeichnet, dass** die Füllöffnung (14) und die Ansaugöffnung (11) in Längsrichtung versetzt am Zylinder (7) angeordnet sind.

7. Dosiergerät (1) gemäss einem der Ansprüche 5 oder 6, **dadurch gekennzeichnet, dass** die Füllöffnung (14) mit einem Kopplungsmittel zum Ankoppeln einer Fluidquelle, insbesondere einer Luer-Kopplung, in Fluidkommunikation bringbar ist, wobei mit Vorteil eine Ventilvorrichtung fluidmässig zwischen der Füllöffnung (14) und dem Kopplungsmittel angeordnet ist, bevorzugt ein Schnabelventil, welches die Füllöffnung (14) gegen einen Fluidstrom von der Fördervorrichtung (5) nach aussen sperrt.

8. Dosiergerät (1) gemäss einem der Ansprüche 1 bis 7, **dadurch gekennzeichnet, dass** die Fördervorrichtung (5) zumindest zwei Ansaugöffnungen (11, 11') umfasst, welche in Längsrichtung versetzt am Zylinder (7) angeordnet sind.

9. Dosiergerät (1) gemäss Anspruch 8, **dadurch gekennzeichnet, dass** jede Ansaugöffnung (11, 11') mit dem Innenraum (3, 3') eines separaten, ihr zugeordneten zumindest teilweise kollabierbaren, Behälters (2, 2') in Fluidverbindung bringbar ist.

10. Dosiergerät (1) gemäss Anspruch 8, **dadurch gekennzeichnet, dass** zumindest zwei Ansaugöffnungen (11, 11') jeweils mit einem separat abgetrennten Innenraum (3, 3') eines Behälters (2) in Fluidverbindung bringbar sind, wobei die separat abgetrennten Innenräume (3, 3') derart ausgestaltet sind, dass wenigstens zwei Fluide voneinander getrennt in dem Behälter (2) aufnehmbar sind.

11. Dosiergerät (1) gemäss einem der Ansprüche 1 bis 10, **dadurch gekennzeichnet, dass** der zumindest eine Behälter (2) zusätzlich ein Verschlussstück (23) umfasst, wobei das Verschlussstück (23) insbesondere fest im Behälter (2) angeordnet und über einen Anschlusskanal (24) mit der Fördervorrichtung (5) in Fluidkommunikation bringbar ist.

12. Dosiergerät (1) gemäss einem der Ansprüche 1 bis 11, **dadurch gekennzeichnet, dass** die Fördervorrichtung (5) zusätzlich eine Analytiköffnung (15) umfasst, welche in Längsrichtung zu der Ansaugöffnung (11) oder den Ansaugöffnungen (11, 11') und der Auslassöffnung (12) versetzt am Zylinder (7) angeordnet und mit einer Analytikvorrichtung in Fluidverbindung bringbar ist.

13. Dosiergerät (1) gemäss einem der Ansprüche 1 bis 12, **dadurch gekennzeichnet, dass** der erste Kolben (9) von einem ersten Förderantrieb (4) und der zweite Kolben (10) von einem zweiten Förderantrieb (4') antreibbar ist.

14. Dosiergerät (1) gemäss einem der Ansprüche 1 bis 12, **dadurch gekennzeichnet, dass** der erste Kolben (9) und der zweite Kolben (10) von einem gemeinsamen Förderantrieb (4) antreibbar sind.

15. Dosiergerät (1) gemäss Anspruch 14, **dadurch gekennzeichnet, dass** zumindest ein Kolben indirekt mit dem Förderantrieb (4) in Wirkverbindung bringbar ist.

16. Dosiergerät (1) gemäss Anspruch 15, **dadurch gekennzeichnet, dass** ein indirekt mit dem Förderantrieb (4) in Wirkverbindung bringbarer Kolben (9) über ein, vorzugsweise in seinem inneren angeordnetes, Federelement (40) mit dem Förderantrieb (4) in Wirkverbindung bringbar ist.

17. Dosiergerät (1) gemäss einem der Ansprüche 1 bis 16, **dadurch gekennzeichnet, dass** der erste Kolben (9) und der zweite Kolben (10) einstückig ausgebildet und über einen Faltenbalg (34) miteinander verbunden sind.

18. Dosiergerät (1) gemäss einem der Ansprüche 1 bis 17, **dadurch gekennzeichnet, dass** zumindest ein Förderantrieb (4) als Spindelantrieb ausgebildet ist.

19. Dosiergerät (1) gemäss einem der Ansprüche 1 bis 18, **dadurch gekennzeichnet, dass** zumindest ein Förderantrieb (4) ein Kurvengetriebe (18) umfasst.

20. Dosiergerät (1) gemäss einem der Ansprüche 1 bis 19, **dadurch gekennzeichnet, dass** das Dosiergerät (1) eine Injektionsvorrichtung zur vorzugsweise kontinuierlichen subkutanen Abgabe eines Fluids an einen Patienten umfasst.

## Claims

1. Dosing device (1) for dispensing a fluid under aseptic conditions, comprising at least one container (2) with an interior (3), and a conveying device (5) which is driven by at least one conveying drive (4) in order to convey the fluid out of the interior (3) of the at least one container (2), wherein the fluid is conveyable by means of the conveying device (5) from the container (2) to a dispensing opening (6), wherein the conveying device (5) comprises a cylinder (7), with at least one intake opening (11) and at least one outlet opening (12) on a cylinder inner wall (8), and also a first piston (9) and a second piston (10), wherein the first piston (9) and the second piston (10) are mounted inside the cylinder (7) in such a way as to be displaceable in the longitudinal direction, and wherein the first piston (9) and the second piston (10), between the end faces thereof and together with a portion of the cylinder inner wall (8), delimit a variable fluid volume (17), **characterized in that** the dosing device (1) comprises a drive module and a dispensing module which are configured so as to be mutually connectable and separable by a user, wherein the drive module comprises at least parts of the conveying drive (4), in particular optionally a rotary drive, and/or optionally an application drive of an injection device, and the dispensing module has at least the container (2) and also the conveying device (5) and optionally the injection device, and the container (2) is at least partially collapsible.

2. Dosing device (1) according to Claim 1, **characterized in that** the intake opening (11) and the outlet opening (12) are disposed on the cylinder (7) in a manner offset in the longitudinal direction.

3. Dosing device (1) according to either of Claims 1 and 2, **characterized in that** the intake opening (11) is fluidically connectable to the interior (3) of a container (2), and the outlet opening (12) is fluidically connectable to the dispensing opening (6).

4. Dosing device (1) according to one of Claims 1 to 3, **characterized in that** the conveying device (5) has a pressure sensor (13) which is preferably disposed on the cylinder (7), in particular in the longitudinal direction at the level of the outlet opening (12).

5. Dosing device (1) according to one of Claims 1 to 4, **characterized in that** the conveying device (5) additionally comprises a filling opening (14) which is fluidically connectable to the intake opening (11) by displacement of the first piston (9) and of the second piston (10) inside the cylinder (7).

6. Dosing device (1) according to Claim 5, **characterized in that** the filling opening (14) and the intake opening (11) are disposed on the cylinder (7) in a manner offset in the longitudinal direction.

7. Dosing device (1) according to either of Claims 5 and 6, **characterized in that** the filling opening (14) is fluidically connectable to a coupling means, in particular a Luer coupling, for the coupling of a fluid source, wherein advantageously a valve device, preferably a duckbill valve, which blocks the filling opening (14) towards the outside in relation to a fluid stream from the conveying device (5), is disposed in fluid communication between the filling opening (14) and the coupling means.

8. Dosing device (1) according to one of Claims 1 to 7, **characterized in that** the conveying device (5) comprises at least two intake openings (11, 11'), which are disposed on the cylinder (7) in a manner offset in the longitudinal direction.

9. Dosing device (1) according to Claim 8, **characterized in that** each intake opening (11, 11') is fluidically connectable to the interior (3, 3') of a separate and at least partially collapsible container (2, 2') assigned to it.

10. Dosing device (1) according to Claim 8, **characterized in that** at least two intake openings (11, 11') are each fluidically connectable to a separately demarcated interior (3, 3') of a container (2), wherein the separately demarcated interiors (3, 3') are configured in such a way that at least two fluids are receivable separately from each other in the container (2).

11. Dosing device (1) according to one of Claims 1 to 10, **characterized in that** the at least one container (2) additionally comprises a closure piece (23), wherein the closure piece (23) is in particular disposed fixedly in the container (2) and is fluidically connectable to the conveying device (5) via an attachment channel (24).

12. Dosing device (1) according to one of Claims 1 to 11, **characterized in that** the conveying device (5) additionally comprises an analysis opening (15) which is disposed on the cylinder (7), in a manner offset in the longitudinal direction in relation to the intake opening (11) or the intake openings (11, 11') and to the outlet opening (12), and which is fluidically connectable to an analysis device.

13. Dosing device (1) according to one of Claims 1 to 12, **characterized in that** the first piston (9) is drivable by a first conveying drive (4), and the second piston (10) is drivable by a second conveying drive (4').

14. Dosing device (1) according to one of Claims 1 to 12, **characterized in that** the first piston (9) and the second piston (10) are drivable by a common conveying drive (4).

15. Dosing device (1) according to Claim 14, **characterized in that** at least one piston is operatively connectable to the conveying drive (4) in an indirect manner.

16. Dosing device (1) according to Claim 15, **characterized in that** a piston (9) that is operatively connectable to the conveying drive (4) in an indirect manner is operatively connectable to the conveying drive (4) via a spring element (40), which is preferably disposed in the interior of said piston (9).

17. Dosing device (1) according to one of Claims 1 to 16, **characterized in that** the first piston (9) and the second piston (10) are formed integrally and are connected to each other via a bellows (34).

18. Dosing device (1) according to one of Claims 1 to 17, **characterized in that** at least one conveying drive (4) is configured as a spindle drive.

19. Dosing device (1) according to one of Claims 1 to 18, **characterized in that** at least one conveying drive (4) comprises a cam gear (18).

20. Dosing device (1) according to one of Claims 1 to 19, **characterized in that** the dosing device (1) comprises an injection device for preferably continuous subcutaneous dispensing of a fluid to a patient.

## Revendications

1. Appareil de dosage (1) destiné à la distribution d'un fluide dans des conditions aseptiques, comprenant au moins un récipient (2) avec un espace intérieur (3) et un dispositif de transport (5) entraîné par au moins un entraînement de transport (4) pour le transport du fluide hors de l'espace intérieur (3) dudit au moins un récipient (2), dans lequel le fluide peut être transporté au moyen du dispositif de transport (5) du récipient (2) à un orifice de distribution (6), dans lequel le dispositif de transport (5) comprend un cylindre (7) avec au moins une ouverture d'aspiration (11) et au moins une ouverture de sortie (12) dans une paroi intérieure de cylindre (8), ainsi qu'un premier piston (9) et un second piston (10), dans lequel le premier piston (9) et le second piston (10) sont montés à l'intérieur du cylindre (7) de façon déplaçable en direction longitudinale et dans lequel le premier piston (9) et le second piston (10) limitent entre leurs faces frontales de concert avec une partie de la paroi intérieure de cylindre (8) un volume de fluide variable (17), **caractérisé en ce que** l'appareil de dosage (1) comprend un module d'entraînement et un module de distribution, qui peuvent être reliés et séparés l'un de l'autre par un utilisateur, dans lequel le module d'entraînement comprend au moins des parties de l'entraînement de transport (4), en particulier le cas échéant un entraînement rotatif, et/ou le cas échéant un entraînement de placement d'un dispositif d'injection, et le module de distribution présente au moins le récipient (2) ainsi que le dispositif de transport (5) et le cas échéant le dispositif d'injection, et le récipient (2) peut au moins partiellement s'affaisser.

2. Appareil de dosage (1) selon la revendication 1, **caractérisé en ce que** l'ouverture d'aspiration (11) et l'ouverture de sortie (12) sont disposées sur le cylindre (7) de façon décalée en direction longitudinale.

3. Appareil de dosage (1) selon une des revendications 1 ou 2, **caractérisé en ce que** l'ouverture d'aspiration (11) peut être mise en communication fluidique avec l'espace intérieur (3) d'un récipient (2) et l'ouverture de sortie (12) peut être mise en communication fluidique avec l'orifice de distribution (6).

4. Appareil de dosage (1) selon l'une quelconque des revendications 1 à 3, **caractérisé en ce que** le dispositif de transport (5) présente un capteur de pression (13), qui est disposé de préférence sur le cylindre (7), en particulier à hauteur de l'ouverture de sortie (12) en direction longitudinale.

5. Appareil de dosage (1) selon l'une quelconque des revendications 1 à 4, **caractérisé en ce que** le dispositif de transport (5) comprend en plus une ouverture de remplissage (14), qui peut être mise en communication fluidique avec l'ouverture d'aspiration (11) par déplacement du premier piston (9) et du second piston (10) à l'intérieur du cylindre (7).

6. Appareil de dosage (1) selon la revendication 5, **caractérisé en ce que** l'ouverture de remplissage (14) et l'ouverture d'aspiration (11) sont disposées sur le cylindre (7) de façon décalée en direction longitudinale.

7. Appareil de dosage (1) selon une des revendications 5 ou 6, **caractérisé en ce que** l'ouverture de remplissage (14) peut être mise en communication fluidique avec un moyen de couplage pour le raccordement d'une source de fluide, en particulier un raccord Luer, dans lequel un dispositif de soupape est avantageusement disposé fluidiquement entre l'ouverture de remplissage (14) et le moyen de couplage, de préférence une valve à bec, qui bloque l'ouverture de remplissage (14) contre un écoulement de fluide du dispositif de transport (5) vers l'extérieur.

8. Appareil de dosage (1) selon l'une quelconque des revendications 1 à 7, **caractérisé en ce que** le dispositif de transport (5) comprend au moins deux ouvertures d'aspiration (11, 11'), qui sont disposées sur le cylindre (7) de façon décalée en direction longitudinale.

9. Appareil de dosage (1) selon la revendication 8, **caractérisé en ce que** chaque ouverture d'aspiration (11, 11') peut être mise en communication fluidique avec l'espace intérieur (3, 3') d'un récipient séparé (2, 2') qui lui est associé et qui peut au moins en partie s'affaisser.

10. Appareil de dosage (1) selon la revendication 8, **caractérisé en ce qu'**au moins deux ouvertures d'aspiration (11, 11') peuvent être mises en communication fluidique respectivement avec un espace intérieur séparément séparé (3, 3') d'un récipient (2), dans lequel les espaces intérieurs séparément séparés (3, 3') sont configurés de telle manière qu'au moins deux fluides puissent être contenus séparément l'un de l'autre dans le récipient (2).

11. Appareil de dosage (1) selon l'une quelconque des revendications 1 à 10, **caractérisé en ce que** ledit au moins un récipient (2) comprend en outre une pièce de fermeture (23), dans lequel la pièce de fermeture (23) est disposée en particulier de façon fixe sur le récipient (2) et peut être mise en communication fluidique avec le dispositif de transport (5) au moyen d'un canal de raccordement (24).

12. Appareil de dosage (1) selon l'une quelconque des revendications 1 à 11, **caractérisé en ce que** le dispositif de transport (5) comprend en outre une ouverture d'analyse (15), qui est disposée sur le cylindre (7) de façon décalée en direction longitudinale par rapport à l'ouverture d'aspiration (11) ou aux ouvertures d'aspiration (11, 11') et à l'ouverture de sortie (12), et qui peut être mise en communication fluidique avec un dispositif d'analyse.

13. Appareil de dosage (1) selon l'une quelconque des revendications 1 à 12, **caractérisé en ce que** le premier piston (9) peut être entraîné par un premier entraînement de transport (4) et le second piston (10) peut être entraîné par un second entraînement de transport (4').

14. Appareil de dosage (1) selon l'une quelconque des revendications 1 à 12, **caractérisé en ce que** le premier piston (9) et le second piston (10) peuvent être entraînés par un entraînement de transport commun (4).

15. Appareil de dosage (1) selon la revendication 14, **caractérisé en ce qu'**au moins un piston peut être mis indirectement en liaison active avec l'entraînement de transport (4).

16. Appareil de dosage (1) selon la revendication 15, **caractérisé en ce qu'**un piston (9) pouvant être mis indirectement en liaison active avec l'entraînement de transport (4) peut être mis en liaison active avec l'entraînement de transport (4) par l'intermédiaire d'un élément de ressort (40), disposé de préférence dans son intérieur.

17. Appareil de dosage (1) selon l'une quelconque des revendications 1 à 16, **caractérisé en ce que** le premier piston (9) et le second piston (10) sont réalisés d'une seule pièce et sont reliés l'un à l'autre par un soufflet à plis (34).

18. Appareil de dosage (1) selon l'une quelconque des revendications 1 à 17, **caractérisé en ce qu'**au moins un entraînement de transport (4) est formé par un entraînement à broche.

19. Appareil de dosage (1) selon l'une quelconque des revendications 1 à 18, **caractérisé en ce qu'**au moins un entraînement de transport (4) comprend une commande à cames (18).

20. Appareil de dosage (1) selon l'une quelconque des revendications 1 à 19, **caractérisé en ce que** l'appareil de dosage (1) comprend un dispositif d'injection pour la distribution sous-cutanée de préférence continue d'un fluide à un patient.
